# FASCICULE DE BREVET EUROPEEN

(11) **EP 1 301 791 B1**
(45) Date de publication et mention de la délivrance du brevet: **20.06.2007**
(21) Numéro de dépôt: 01951782.0
(22) Date de dépôt: 10.07.2001
(51) Int. Cl.: G01N 33/558, G01N 33/531, G01N 33/543, G01N 33/542

(54) **DISPOSITIF ET PROCEDE DE DETECTION MOLECULAIRE**
VORRICHTUNG UND VERFAHREN ZUM MOLEKULAREN NACHWEIS
DEVICE AND METHOD FOR MOLECULAR DETECTION

(30) Priorité: 11.07.2000 FR 0009067
(43) Date de publication de la demande: 16.04.2003
(73) Titulaire: Toledano, Jacques, 95870 Bezons (FR)
(72) Inventeur: Toledano, Jacques, 95870 Bezons (FR)
(86) Numéro de dépôt international: PCT/FR2001/002213
(87) Numéro de publication internationale: WO 2002/004915

(56) Documents cités:
- EP-A- 0 279 097
- WO-A-00/00826
- WO-A-97/06439

## Description

L'objet de la presente invention est d'obtenir un signal qualitatif et quantitatif,de la presence,de quelques molecules d'analytes recherchées dans un échantillon quelconque (fluides biologiques,vegetaux ou ecologiques,frottis de muqueuse, biopsie de tissus biologiques ou vegetaux,tumeurs solides, etc). L'extrême sensibilté de la technique qui va être decrite plus loin,permet la detection et le suivi de maladies virales,bacteriennes,cancers,comme tous les autres domaines de la biologie classique mais surtout,elle ouvre la voie à la detection de nouvelles substances,inconnues jusqu'à une periode très recente du fait qu'elles circulaient à des taux indetectables par les methodes classiques tels que automates de laboratoire ou la RIA (Radio immuno assay).Parmi ces substances jusque là inconnues,on peut citer à titre indicatif et non limitatif,les proteomes (produits par un genome modifié dans les OGM : organismes genetiquement modifiés), les nouvelles generations de marqueurs tumoraux (peptides indetectables mais hautement specifiques de cancers (ex :vessie), differents des anciens marqueurs qui etaient peu specifiques et peu fiables , les produits de translation genique spontanée : substances intracellulaires produites par un ADN qui a subi une perte ou un remplacement d'un ou plusieurs acides aminés d'origine hereditaire(cancer du sein ou maladie d'alzheimer),toxique (tabac,pollution,amiante) ou medicamenteuse(suvi du pharmacogenome).Enfin les puces à ADN donnent en quelques secondes des milliers de renseignements d'ordre genetique que seuls des logiciels puissants sauront interpréter : des oncogènes ont été identifié par les puces à ADN ces derniers mois mais la proteine produite par ces oncogènes est indetectable : la presente invention,capable de detecter des traces de proteine de niveau moleculaire aussi vite que la puce à ADN en detecte le gène correspondant accompagnera donc ce progrès.

Il va de soi que si l'affection consiste plutôt en une absence de la proteine ou de l'analyte concernés,cette absence sera aussi detectée (pertes de gènes dans certaines affections ou après certains traitements)

Le terme de proteome,inventé par Friedrich en 1996 (Nat.Biotechnologies 14:1234-1237) est defini par Keith L.Williams et Denis F.Hochstrasser dans leur livre « Proteome Research » (Ed.Springer 1997) : c'est la PROTEine exprimée par un genOME.

Il existe près de 100.000 gènes dans le genome humain qui sera entièrement sequencé vers 2005.Chaque gène code pour une à trois proteines differentes dont la majorité n'est pas encore identifiée en structure ni en fonction.

Des programmes de bioinformatique ont commencé à identifier et repertorier ces proteines et d'ici quelques années des banques de données telle la Database Swiss 2D Page,auront listé la totalité des proteines du corps humain .Il est permis de rêver qu'en face de chaque gène figurera la ou les proteines correspondantes ainsi que les lesions possibles au niveau de chaque gène, mais surtout quelle proteine anormale est alors produite par cette anomalie genique.L'interêt est immense : la moindre anomalie genique sera detectée,identifiée puis ciblée par des therapeutiques adéquates. Comment la detecter sans avoir à sequencer la totalité des gènes du patient concerné? : en detectant son proteome.

R.Strohman (1994) dans Bio/Technology 12 :156-164, estime que seulement 2% des maladies humaines resultent d'un defaut au niveau d'un gène (absence de production d'une protéine ou presence d'une proteine altérée).Il ajoute que la sévérité de ces maladies est fonction du patrimoine genetique de l'individu concemé.C'est le cas du p53,du RB ou WT1 qui sont des gènes de type anti-oncogènes bien identifiés.La perte germinale de l'un des allèles de ces gènes,predispose au cancer ;la perte ulterieure du second allèle provoque l'emergence du cancer.

La plus recente decouverte proteomique concerne les travaux du professeur Ben Zeev de l'Institut Weizmann en Israel, publiés dans Proceedings Natl. Acad. Sciences - 11 Mai 1999 - page 5222: son equipe est parvenue à reconstituer le puzzle conduisant à la cancerisation d'une cellule du colon : une mutation de l'ADN de cette cellule produit un proteome : la beta-cathenine dont le rôle demontré est de stimuler fortement l'expression d'un gène codant pour la cycline D1.Dans la cellule normale,la cycline D1 gère les divisions cellulaires selon un programme harmonieux.L'elevation brutale de cycline D1 va provoquer la proliferation incontrôlée des cellules cancereuses du colon.

Ceci est d'un interêt diagnostic : la detection de betacathenine associée à la detection de l'elevation du taux de cycline D1 signent sans le moindre doute la naissance d'un cancer du colon au stade infra-clinique; mais aussi d'un intérêt thérapeutique : le ciblage therapeutique par des inhibiteurs de la cycline ou de la betacathenine,au stade precoce de la maladie,offrira au patient un confort de vie (sans chimiotherapie ni metastases) et des economies substantielles dans les budgets de la santé ;enfin d'un intérêt de suivi de l'affection : détection de rechutes,evaluation des résultats de chaque traitement pour proposer la drogue ideale,contrôle mensuel à coût très reduit,etc

L'interêt reside egalement en une forte specificité et une forte sensibilité des proteomes produits,ce qui est une nouveauté dans le domaine des marqueurs du cancer.Jusqu'à present,ces derniers n'offraient qu'une faible sensibilité (ne voient leur taux grimper que dans 50 % des cancers) et une faible specificité (taux augmenté alors qu'il n'y a pas de cancer : cas des femmes enceintes,des gros fumeurs,etc).Les proteomes produits par une anomalie genique,n'existent pas chez une personne saine.Leur apparition signe avec certitude l'anomalie genique.

Le problème majeur est qu'ils sont indetectables au stade precoce de l'affection : il n'est plus question ici de nanogrammes (comme pour les marqueurs tumoraux anciens) ou de picogrammes (pour les nouveaux marqueurs plus specifiques ou les cytokines) par milliltre,mais de nombre de molecules par millilitre d'échantillon ou par fragment de biopsie.

Le procédé objet de la presente invention est parvenu à detecter la presence de quelques molecules dans un micro-échantillon.C'est mieux ou aussi sensible que les techniques d'hybridation par la Polymerase Chain Reaction mais c'est infiniment plus simple,plus rapide (résultat instantané) et moins coûteux que la PCR.

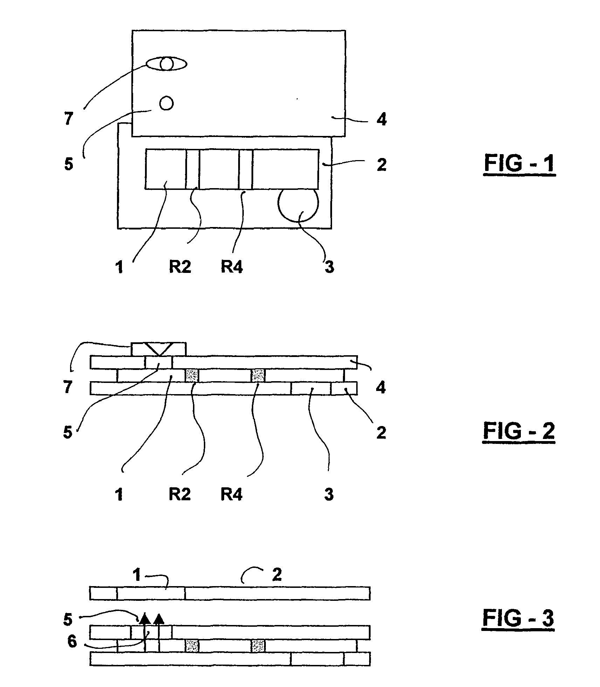

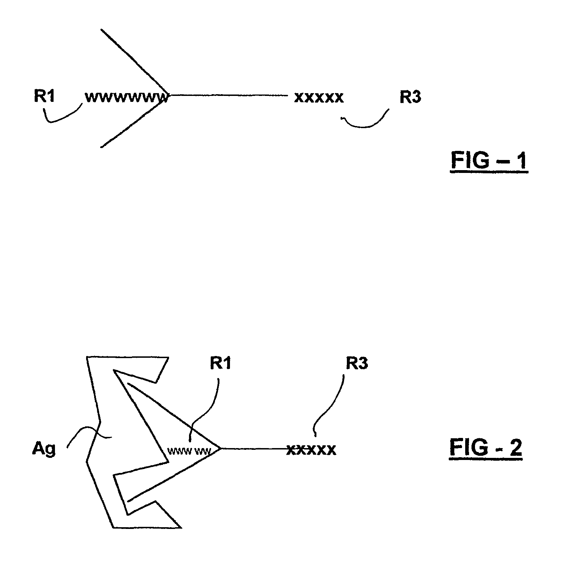

### DESCRIPTION DU DISPOSITIF(Planche I Figures 1 et 2)

Un filtre (1) en materiel poreux quelconque (papier, cellulose, nylon, nitrocellulose, acetate de cellulose,etc) est preparé selon le mode decrit plus loin puis inseré entre :
D'une part un support superieur (4) plat rigide perforé d'un trou (5) de quelques millimetres de diametre,supportant un entonnoir (7) destiné à recevoir l'échantillon et les fluides du test qui vont migrer à l'interieur du filtre.
Et d'autre part,un support inferieur (2) perforé à l'extrémité opposée d'un trou (3) de plusieurs millimetres de diametre. La face inferieure du filtre à cet endroit est imbibée de réactifs (substrat chromogène si le traceur est un enzyme par exemple) destinés à se colorer pour emettre un signal visible à travers le trou 3 en retournant le dispositif à la fin du test.

L'ensemble sera de preference emballé en milieu opaque,sous vide avec un dessicant.

### DESCRIPTION DU PROCEDE

### Principe : (Planche II - Figures 1 et 2)

en prenant comme exemple un système de détection d'un antigène.

Plusieurs molecules d'un réactif (R1) sont fixés à l'endroit precis (sites NH2 ou epitopes) d'un anticorps servant à la detection d'un antigène dans l'échantillon testé alors que plusieurs molecules d'un autre réactif (R3) sont fixées à l'autre extrémité de l'anticorps (site Fc).

Planche III Figures 1 et 2 :Les molecules de réactifs R1 peuvent se lier instantanément à des molecules d'un réactif R2 correspondant,solidement (liaison covalente) fixé sur le filtre,immobilisant ainsi sur place l'anticorps en absence d'antigène.

Si l'anticorps rencontre dans l'échantillon,l'antigène (Ag) recherché,ce dernier masque,occulte le réactif R1 (Planche II - Fig 2) qui ne se liera donc pas au réactif R2 : l'anticorps va alors continuer sa migration dans le filtre.

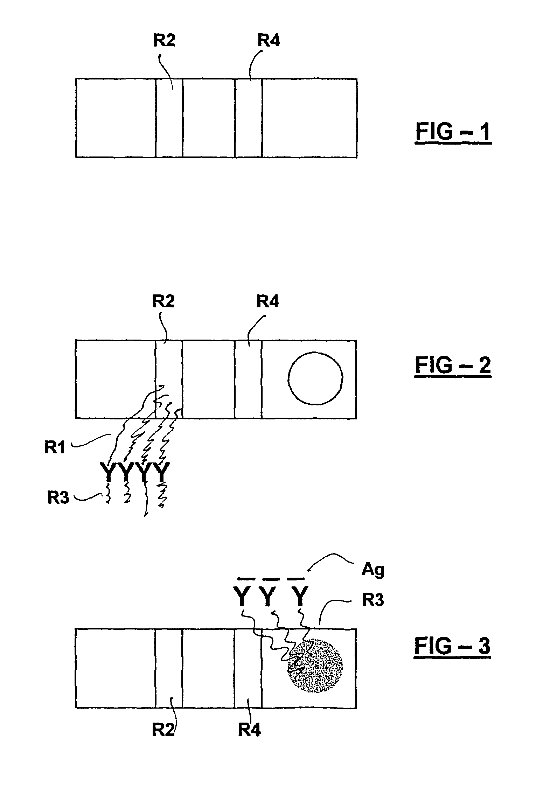

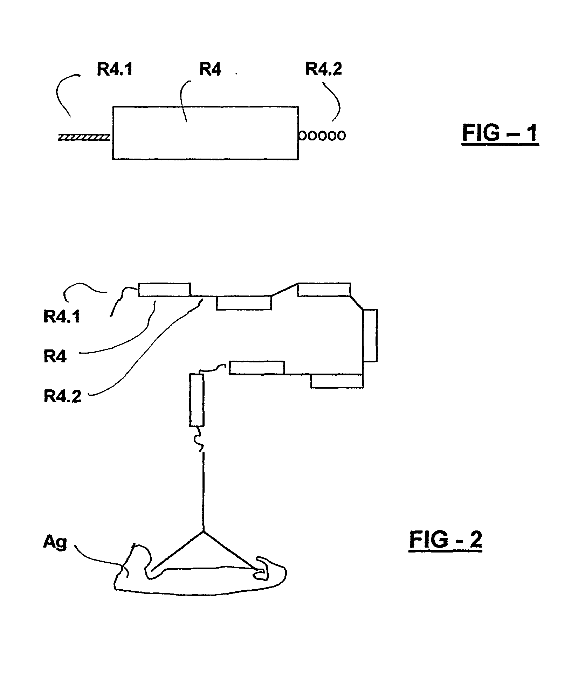

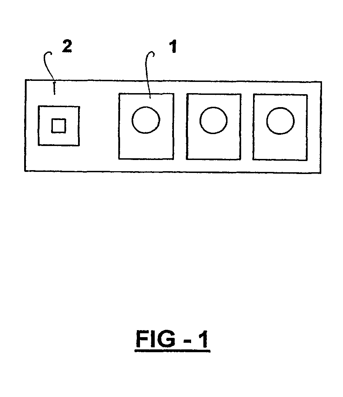

Un peu plus loin sur ce dernier,est disposée une ligne de d'une solution contenant un reactif R4 lié à plusieurs molecules traçantes T (une enzyme T-enz si le systeme est immunoenzymatique).Ce reactif R4 est capable de se lier instantanément avec le réactif R3 (fixé sur le site Fc de l'anticorps ayant echappé au réactif R2) pour migrer vers l'extrémité du filtre. Les nombreuses molecules de traceur fixées sur R3,lui même emporté par R4 vont declencher une serie de nombreuses reactions dont le produit final va reveler un substrat spécifique : si le traceur est une enzyme (T-enz) le substrat sera un chromogène.Planche III - Figure 3

Si ce traceur est fluorescent,la reaction avec le substrat devra être lue aux rayons UV,si le traceur est radioactif il devra être mesuré par un compteur dans la zone-signal.

Le signal ne sera donc plus proportionnel au nombre d'anticorps qui se sont liés à un antigène,mais au nombre de molecules de produit final produites et reagissant avec le substrat.

Un exemple decrit dans le mode operatoire ci dessous,illustre le procédé : des groupements R1 et R3 (qui ne sont pas des traceurs) (Planche Il - Figure 1) sont fixés sur un anticorps monoclonal puis filtrés par chromatographie ;des groupements R2 sont fixés au debut du filtre et un reactif R4 supportant plusieurs molecules de traceur est deposé plus loin sur ce filtre.(Planche III - Figure 1)

La gouttelette d'échantillon est deposée en (5) sur le filtre (1) au fond de l'entonnoir (7).Puis on depose une solution d'anticorps supportant R1 et R3.Si l'échantillon est negatif (absence d'antigène) R1 (Planche III - Figure 2) est piégé par R2.Le réactif R3 n'ira donc pas decrocher R4 et les fluides arrivant dans la zone chromogène ne donneront aucune coloration.

Si l'échantillon est positif,le groupement R1 est occulté spatialement par la molecule d'antigène, echappe donc à R2,continue à migrer avec les fluides.Le groupement R3,vient decrocher l'ensemble traceurs + réactif R4 pour aller colorer la zone chromogène.(Planche III - Figure 3)

### DOSAGES SEMI-QUANTITATIFS

Principe : il s'agit de colorer la zone signal si le taux d'antigène est supérieur au taux normal, Methode adoptée : L'échantillon n'est plus deposé directement dans le filtre mais dans un petit tube associé au dispositif et contenant des anticorps non preparés (pas de R3 notemment pour ne pas decrocher de R4) en quantité capable de se lier à la quantité d'antigène limite de la normale,les antigènes eventuellement en excès resteront donc libres dans cette solution qui va alors être deposée sur le filtre. Les anticorps normalement preparés sont ensuite deposés dans le filtre.

Hypothèse normale : Il n' y a pas d'excès d'antigène : les premiers anticorps (sans R3) complexés aux antigènes dans la première etape,migrant avec les seconds anticorps dûment preparés (R1+R3) ne decrochent pas les groupes traceurs + R4.Les seconds anticorps,n'ayant plus trouvé d'antigène,sont arrêtés par R2 et ne donneront pas de coloration.Hypothèse pathologique : excès d'antigène : tous les anticorps (ceux supportant R3 et ceux sans R3) sont saturés : le système fonctionne alors comme precedemment decrit : les anticorps portant R3 echappent aux reactifs R2 fixés sur le filtre,decrochent les groupes traceurs+R4 et va provoquer plus loin la coloration du signal.

### ORIGINE DE LA GRANDE SENSIBILITE DU PROCEDE :

Le réactif R4 (Planche IV Figure 1) supportant plusieurs molecules de traceur (enzyme si système immunoenzymatique) est deposé simplement sur le filtre en aval de la zone R2.Ce reactif R4 est conçu pour se lier rapidement au réactif R3 des anticorps qui ont echappé aux réactifs R2 du filtre en amont.C'est une liaison cette fois-ci covalente, qui va decoller du filtre l'ensemble traceurs + R4.Mais un anticorps dont le site Fc ne supporte qu'une molecule de R3,ne decrochera qu'une molecule de R4+traceurs.Pour augmenter la sensibilité,il faudrait que la même molecule d'anticorps decroche plusieurs molecules de R4. Pour cela,un travail chromatographique complexe permet de fixer plusieurs molecules de R3 sur le même anticorps.(Planche IV - Figure 2).Ce qui fait que pour une molecule d'antigène trouvée par un anticorps,ce dernier va entrainer derrière lui un cortège de dizaines de molecules de traceur dont le produit final va arriver à la zone-signal pour y delivrer une puissante coloration du substrat chromogène.(Planche IV - Figure 2).

Le système complet de réactifs utilisés est identique quelque soit l'affection recherchée.Seul variera l'anticorps deposé à la suite de l'échantillon,puisqu'il sera specifique de ce qu'on recherche. Le procédé est donc universel en ce sens que les deux cartes enserrant le filtre seront identiques d'un test à l'autre. Une ampoule en polypropylène,bien etiquettée,contenant la solution d'anticorps specifique, accompagnera dans un emballage separé,le dispositif et elle sera differente d'un test à l'autre. Enfin,ce système,contrairement aux differents predecesseurs de l'art anterieur,donne une reponse quasi instantanée (quelques secondes) - Par comparaison,le système Elisa met 3 heures,l'immuno-PCR : 3 jours

L'exemple choisi pour expliquer le mecanisme du procédé concerne donc une detection immunologique d'un antigène par un anticorps.Mais ce procédé s'applique en fait à toute reaction mettant en jeu la reconnaissance d'une molecule d'analyte par un ligand : détection de brins d'ADN par sondes marquées par un réactif du type R1, detection de recepteurs membranaires par la cytokine correspondante,detection d'une sous-unité de protéine par la sous-unité correspondante,etc.Le procédé decrit n'est donc pas limitatif. De plus, à l'interieur d'une même application,un grand nombre de variantes apportent chacune un avantage selon les necessités de la detection.Elles seront decrites plus loin mais il pourra s'agir par exemple d'amplifier encore plus la reaction colorimetrique du substrat : le traceur peut provoquer la formation d'un premier composé (X1) en un turn over rapide (1000 molecules par seconde) qui réagirait avec une seconde ligne supportant un réactif R5 pour produire 1000 molecules par seconde d'un deuxième composé (X2) à partir d'une molecule de X1.Ce sera finalement 1.000.000 de molecules de X2 qui vont colorer le substrat et non la molecule de traceur + R4 qui les a engendrés en amont.

Sachant que le substrat chromogène est preparé de telle sorte qu'il reagisse fortement et très vite (concentration elevée des composants du substrat), ou que des appareillages de mesure extrêmement performants existent (rayon laser identique à celui des lecteurs de glycemie ou electrode numerique de type pH mètre,etc) il apparaît donc que ce système atteint une sensibilité exceptionnelle.

### DETECTION D'ANTICORPS SERIQUES

Le procédé est identique sauf que les marquages R1 et R3 se font sur la molecule d'antigène dont on recherche l'anticorps correspondant dans l'échantillon.Si elle detecte cet anticorps,elle echappera dans le filtre,au réactif R2(le réactif R1 etant occulté) et elle ira réagir par l'intermediaire de son réactif R3 avec le réactif R4 du filtre pour produire le signal plus loin.Un exemple de detection d'anticorps est decrit plus loin.

### L'ART ANTERIEUR

Le brevet français No 90-12661 (PCT/FR91/00891) decrit une phase selective universelle capable de pieger tout anticorps marqué de souris par l'intermediaire d'immunoglobulines anti-idiotypes de souris,lorsque l'anticorps ne s'est pas complexé à un antigène.Alors que les complexes antigène-anticorps marqués migrent vers la zone signal.

C'est un dispositif immunologique (un anticorps piège un autre anticorps) pur ne faisant pas intervenir le piegeage des anticorps arrivant par des reactfs non immunologiques greffés sur l'anticorps et sur le filtre.D'où ses faibles performances en sensibilité.

Le brevet US 4,948,726 du 14 Aout 1990 (Inventeur : Claude C. Longoria) decrit un dispositif vertical contenant une membrane horizontale chargée electrostatiquement : en versant un melange anticorps-antigène sur cette membrane,ces derniers sont retenus alors que les anticorps seuls traversent la membrane.C'est un système immuno-enzymatique de filtration verticale de faible performance et source de bruits de fond considerables.

WO 0 000 826 divulgue un dispositif et un procédé immunochromato graphique.

Le brevet français No 98/09599 du 28 Juillet 1998 decrit un dispositif d'electrodes separées par un filtre inerte. L'etectrode superieure est perforée pour y deposer l'échantillon puis la solution d'anticorps marqués, l'ensemble anticorps-antigène migrant vers la zone-signal sans être capté par les electrodes,alors que les anticorps sans antigène sont captés par les electrodes.C'est un système immuno-enzymatique (l'anticorps est marqué de façon traditionnelle par une enzyme) performant (detecte le picogramme par ml).

Le procédé objet de la presente invention en diffère par :
1 : le traceur est dans la phase selective et non sur l'anticorps
2 : la phase selective n'est pas constituée d'electrodes mais d'un filtre poreux sur lequel ont été fixés des réactifs qui piègent de façon chimique et covalente les anticorps sans antigène.
3 : la grande difference par rapport au brevet français 98/09599 est que cette phase selective en piegeant l'anticorps quand il est libre d'antigène, entraîne egalement mais plus loin sur le filtre,la non-migration du traceur.
4 : son extrême sensibilité : ici,pour une molecule d'antigène detectée,des dizaines de molecules de traceur parviennent à la zone signal ;ce qui permet de detecter moins du millipicogramme par millilitre d'échantillon alors que le brevet français detectait le picogramme.

L'art anterieur dans la detection de proteomes concerne des techniques très anciennes dont la plus representative est l'electrophorèse bi-dimensionnelle sur gel : un cocktail de proteines migre verticalement à l'intérieur d'un gel sous l'effet d'un champ électrique et les proteines se groupent en spots successifs selon leur poids moleculaire,puis chaque spot migre horizontalement selon le pH pour produire une succession de petits spots.Sachant à l'avance que telle proteine a un tel pH et un tel poids moleculaire connus,elle est attendue à un certain endroit du gel.Le spot est ensuite coloré ou revélé de diverses manières.C'est une methode efficace mais longue,coûteuse et très peu sensible : pour lire un spot à l'oeil nu,il faut des quantités considerables de proteines.

Des ameliorations techniques ont été apportées pour ameliorer la sensibilité : la detection fluorescente s'est avérée plus efficace mais elle parasite les charges et le pH des molecules proteiques marquées.

Hochstrasser et Merril,en 1988,dans Appl.Theor. Electrophor. 1 :35-40 produisent une polymérisation du gel de polyacrylamide pour la detection de proteines par marquage à l'argent.

Jonscher KR,Yates JR 3rd (1997) dans Anal.Biochem 244 :1-15 (The quadrupole ion trap mass spectrometer - a small solution to a big challenge) s'inspirent d'un travail de Wilm et Mann en 1996 (Analytical properties of the nano electrospray ion source - Anal Biochem 66 :1-8) pour analyser des échantillons sousmis à un puissant champ electrostatique pour dissocier par refraction d'un rayon laser,les elements proteiques selon leur charge.

Wilkins MR,Hochstrasser D et Sanchez JC en 1997 (Electrophoresis 18 : 403-408) dans Protein Identification with N- and C- Terminal sequence tags in Proteome projects pratiquent la greffe de fragments d'ADN sur les sequences N et C terminales des proteines recherchées,afin de mieux les visualiser à des doses infinitesimales.

### MODE PREPARATION D'UN DISPOSITIF COMPLET

En prenant comme exemple un système ultrasensible de detection de la proteine de surface du virus de l'hepatite B (Antigène HBs) par un anticorps sur lequel est greffé SMCC (R1) et avidine (R3).Sur le filtre R2 sera SPDP et R4 : Biotine - peorxydase .La revelation untilisera le potentiel Glucose oxydase-D-Glucose = H202 et TMB.Tous ces réactifs sont decrits ci dessous.
A : PREPARATION DE LA SOLUTION D'ANTICORPS :
   A.1 : Greffage du réactif R1
   A.2 : Greffage de R3
   A.3 : Mise en ampoule polypropylène et etiquetage
B : PREPARATION DU FILTRE
   B.1 : Fixation de R2
   B.2 : Depose de l'ensemble Traceurs + R4
   B.3 : Depose du substrat
C : ELEMENTS PHYSIQUES
   C.1 : Carte superieure
   C.2 : L'entonnoir
   C.3 : Carte inferieure
D : MONTAGE - ENSACHAGE - ETIQUETTAGE

### A : PREPARATION DE LA SOLUTION D'ANTICORPS

A.1: L'anticorps est une IgG1 monoclonale de Chèvre dirigée contre la sous-unité Ad de la molecule de surface du virus de l'hepatite B (HBs/Ad de Virostat-USA).Le réactif R1 est le N-succinimidyl 4-(N-maleimidomethyl)cyclohexane-1-carboxylate que nous nommerons par commodité pour la suite : SMCC.

Pour fixer le SMCC sur le site epitopique NH2 de l'anticorps,il faut introduire des groupements thiols (-SH) à cet endroit precis de l'anticorps.il existe de nombreux acides aminés à la surface de toute la molecule d'IgG possedant des liaisons disulfures -S-S-

Au niveau du site NH2 seul un residu Cystine possède une liaison -S-S-

C'est donc à ce niveau qu'il faut provoquer un clivage (formation d'un groupe sulfhydryl libre) pour y fixer la molecule de SMCC et pas ailleurs. Pour cliver le pont disulfure -S-S- du residu cystine du site NH2 on incube pendant 2 Heures à 37°C 100 microlitres (10 mg/ml) de la solution d'anticorps dans 10 mM 2-mercaptoethylamine in 0,1 M sodium phosphate buffer , pH 6,0 et contenant 5mM d'EDTA.

Après 20 minutes d'incubation à temperature ambiante,l'on confirme la presence de groupes thiols -SH en mesurant l'adsorbance à 324 nm utilisant une absorption molaire de 19.800 mol-1.cm-1.

Il existe de nombreuses autres methodes de reduction-clivage de residus cystine (DTT ou S-acetylmercaptosuccinic anhydre prealablement dilué dans N,N-dimethylformamide pendant 30 minutes à temperature ambiante) .Celle retenue ici est de loin la meilleure car elle ne provoque pas de polymerisation des anticorps et le caractère monomerique est ici indispensable (sinon chute dramatique de la sensibilité : seuls quelques sites NH2 seront libres) Elle s'inspire de Ishikawa, Yoshitake, Imagawa, Sumiyoshi (Ann. N.Y. Acad.Sci. U.S.A. ,78,5807,1981

Le réactif R1 (SMCC) est ensuite mis en contact avec la solution d'anticorps preparée ci dessus en 0,1 M Sodium phosphate buffer pH6 contenant 2,5 mM EDTA à 4°C pendant 20 heures ou à 30°C pendant 1 heure. Cette solution est ensuite purifiée sur LKB Ultrogel AcA 44 pour eliminer les anticorps qui n'ont pas fixé de SMCC et les molecules libres de SMCC.

### A.2 GREFFAGE DE R3. Exemple choisi : R3 : AVIDINE

A.2.1 : Thiolation de l'avidine : en solution de S-acetyl mercaptosuccinic anhydre dissous en N,N-dimethylformamide et PBS 0.2 M pH7 30 minutes à 30°C. Les groupes thiols sont alors generés par 0,2 M hydroxylamine - HCl et 5mM EDTA pH7 : rendement : 1,6 groupes thiols par molecule d'avidine:.Elle est choisie ici car elle ne réagit qu'avec la biotine (en R4) et pas avec le SMCC deja fixé sur l'anticorps.
A.2.2 Mise en contact avec l'anticorps (site COOH) et passage rapide en colonne preparative LKB Ultrogel pour eliminer l'avidine restée libre.
A.3 La solution d'anticorps ainsi preparée avec R1 et R3 est diluée dans une solution de tampon,d'antibiotiques,antifongiques, de protéine ballast type BSA 1 mg/ml pour atteindre la dose necessaire pour le test (5 nanogrammes/ml)

### B- PREPARATION DU FILTRE

Le filtre est un materiau poreux quelconque,inerte : cellulose, hydroxycellulose, papier,etc Il est possible d'utiliser des materiaux poreux plus nobles : nitrocellulose,acetate de cellulose,nylon,etc qui ont une porosité beaucoup plus petite mais pour lesquels il faut terminer la preparation du filtre par un blocking proteique (BSA,gelatine) ou chimique (PVP,PEG).

Dans ce modèle,le filtre est un rectangle de cellulose de 30 mm de long sur 12 mm de large et 0,7 mm d'épaisseur.

### B1 : DEPOSE DE R2.

Il s'agit du seul produit reagissant avec R1 (non masqué par antigène) pour immobiliser l'anticorps en cas de resultat negatif. ; le SPDP : (N-succinimidyl 3-(2-pyridyldithio) propionate.Pour le fixer sur le filtre qui ne contient pas de radical chimique réactif,il faut le lier à une protéine presentant un groupe sulfhydryl SH (elles sont nombreuses : ricine,gelatine,etc).La fixation au filtre se fera en solution à chaud.Après refroidissement la protéine est immobilisée et insoluble dans le filtre.Le depôt de R2 se fera sur le filtre à 5 mm en aval du lieu où la solution d'anticorps réagira avec l'échantillon avant qu'elle ne migre vers le lieu de depose de R2.En l'absence d'antigène dans l'échantillon, R1 (SMCC) sera piégé par R2 (SPDP).Par contre en presence d'antigène,R1 sera occulté et neutralisé,ne se liera pas à R2 et l'anticorps poursuivra sa migration vers le groupe traceurs + R4

### B2: DEPOSE DE R4 :

Il s'agit d'une solution de Peroxydase Biotine preparée de la façon suivante :
B2.1 Biotinylation de la peroxydase par Biotinyl-N-hydroxysuccinimide ester
B2.2 : puis bain de 0,2% v/v de glutaraldehyde en 0,1 M PBS pH6,8 pendant 2H à temperature ambiante.

Le rapport Biotine/Peroxydase est de 1/4

Pour proteger la peroxydase on ajoute des ions (ZnCl2 - MgCl2 - CaCl2 0,01 M de chaque,des antibiotiques,un ballast proteique (serum de veau foetal 10 mg/ml) et un anti trypsine-chymotrypsine : l'aprotinine. Dans ce melange,la peroxydase survit sous vide,au noir,plus de deux ans à temperature ambiante..

La solution est deposée à froid,à 5 mm en aval de R2 sous forme d'un trait sur le filtre dans le sens de la largeur,sur la route des fluides arrivants.Elle sera delicatement sechée à 25-30°C

Si les fluides qui arrivent de R2 ne contiennent pas d'anticorps (puisque piegé par R2 en amont),aucun réactif ne se liera à la biotine et la peroxydase ne se decrochera pas.

Si les anticorps arrivent,le réactif R3 (avidine) hautement specifique de la biotine,se lie à la biotine et entraine les molecules de peroxydase avec elle. En fait une molecule d'avidine peut se lier à de nombreuses molecules de biotine elle même liée à plusieurs molecules de peroxydase ce qui augmente la sensibilité.

Une variante permet d'amplifier considerablement la sensibilité : après avoir biotinylé la peroxydase,on y fixe à nouveau,mais en moindre quantité et dans des conditons differentes (tampon,pH,etc),des molecules d'avidine de telle sorte qu'une molecule de peroxydase possède un pôle biotine et un pôle opposé avidine.Ce qui permet au groupe (peroxydase - biotine fixée à R3 - avidine libre) d'entrainer par ce pôle avidine libre,un nouveau groupe biotine-peroxydase-avidine et ainsi de suite pour former un cortège ou une polymerisation lineaire de très nombreuses molecules de traceurs + R4 qui vont colorer le substrat pour une seule molecule d'antigène detectée par l'anticorps.

### B3 : DEPOSE DU SUBSTRAT

A 5 mm en aval de R4+traceur,on depose un trait de Glucose oxydase 500 Ul/ml dans le sens de la largeur.Puis un mm encore en aval,un trait de D-Glucose 1M puis à 5 mm en aval on dépose à la pipette 10 µl de 3-3'-5-5' Tetra methyl benzidine (TMB) à 100 mg/ml.(cette dépose de 10 µl forme un rond de TMB de 8 mm de diametre sur la face inferieure du filtre) Le melange peroxydase (quand les anticorps passent) + glucose oxydase + D-Glucose aboutit à la formation d'ions H+ en très grande quantité qui colorent le TMB en bleu

### C : ELEMENTS PHYSIQUES

### C.1. : ELEMENT SUPERIEUR (4) : Planche I Fig 1 -2

Il s'agit d'un materiau rigide quelconque (carton etanche,plastique PVC,etc). Il sera recouvert à sa face inferieure d'un adhesif double face très fin puis perforé d'un trou de 4 mm de diametre

Cet element superieur vient recouvrir le filtre de telle sorte que le trou soit à quelques mm du bord proximal du filtre,au milieu de la largeur.

### C.2. : L'ENTONNOIR (7) Planche I Figure 1

Est un element en plastique moulé de 10 mm de diametre et 1 mm d'epaisseur avec un trou de 4 mm de diametre en son milieu. Il sera collé sur l'element superieur de telle sorte que son trou corresponde bien au trou de l'element supérieur.

### C.3 : L'ELEMENT INFERIEUR (2) Figure 1 et 2

A partir du même materiau rigide,sa face superieure cette fois,est recouverte de l'adhesif double face et perforé d'un trou de 6 mm de diametre en regard de l'extrémité distale du filtre,à l'endroit precis où sera deposé le TMB.Un carré de plastique transparent de 10mmX10mm vient recouvrir ce trou afin que le filtre ne reste pas à l'air (risque de coloration spontanée du TMB) En retournant le dispositif à la fin du test,on pourra voir le resultat à travers cet orifice.

Il n'est pas souhaitable que cet orifice soit sur l'element supérieur car certains tests demanderont des filtres beaucoup plus courts : le trou de dépose risquera alors d'être trop près de l'orifice du signal : de nombreux lecteurs,à l'image des lecteurs de glycemie,ont une source lumineuse (laser) qui lit le signal par sa face inferieure alors que la goutte de sang a été deposée sur la face superieure.

D'autre part,après la dépose de l'échantillon et des réactifs,il est preferable de couvrir cette face supérieure par un adhesif ou en la remettant dans un etui afin d'eviter tout contact avec des elements dangereux de l'échantillon.Si le trou du signal etait sur cette face superieure,on ne pourrait plus le lire alors que la face inferieure,non recouverte laissera visible le trou du signal qui est de toutes façons protégé par le carré de plastique

Dans une configuration avantageuse,les elements superieur et inferieur peuvent avoir les dimensions d'une carte de credit.

Une autre configuration est très interessante : le filtre est enserré entre deux bandes longues par exemple de 50 mm sur 15 mm de large. Il n'y a pas d'entonnoir.Le patient dépose sa gouttelette d'échantillon sur le filtre puis plonge ce dernier dans un tube contenant la solution d'anticorps qui sera ainsi aspirée par l'orifice de la face superieure.

Dans une autre configuration particulièrement avantageuse,le filtre peut être triangulaire avec une zone signal au contact seulement du sommet du triangle : l'avantage réside en la migration tout à fait différente de molecules ayant un fort pouvoir agregant,une fois parvenues au sommet du filtre triangulaire.

Dans une autre configuration pratique l'echantillon n'est plus deposé à la surface superieure du filtre à travers un trou de l'element superieur mais plutôt à l'extrêmité proximale,laissée libre du filtre : l'operateur aspire une gouttelette d'echantillon par cette extrêmité proximale du filtre puis aspire une goutte de reactif correspondant à la substance recherchée.Cette configuration est aussi valable pour des filtres rectangulaires que des filtres triangulaires.

### D: MONTAGE

On pose l'element inferieur sur un plan de travail.Puis un carré de plastique transparent fin de 10X10 mm sur le trou de 6 mm de diametre afin que le filtre qui va être deposé dessus ne soit pas à l'air.Puis le filtre avec la zone imbibée de TMB bien en regard du trou-signal.

Comme le filtre a une epaisseur de 0,7 mm il est preferable d'ajouter un element intermediaire rigide et perforé de 30X12 mm afin que l'element superieur qui vient se coller au filtre ne soit gondolé sur les bords.

Dans une configuration très avantageuse,la solution d'anticorps peut être integrée au dispositif : Planche 1 - Figure 3 : un reservoir de solution d'anticorps (1) est integré à un couvercle (2).Après la depose de l'échantillon dans le trou (5) du dispositif initial,on rabat le couvercle.Un système d'autoperforation (6)à la fermeture provoque la vidange du reservoir (1) ce qui evite d'utiliser une pipette ou de manipuler des solutions d'anticorps.Si l'échantillon est du sang total prelevé à un doigt,il suffira de déposer la goutte de sang et de fermer pour voir le résultat.

ENSACHAGE dans un sachet plastique noir ou aluminium,opaque,etanche avec une plaque de dessicant de quelques cm2 qui recouvre le dispositif avant la soudure du sac.

ETIQUETTAGE prealable du dispositif,du sachet. Impression d'un mode d'emploi et des precautions d'usage.

L'ensemble ainsi formant une trousse.

### MODE OPERATOIRE

En gardant l'exemple de la detection de l'antigène HBs du virus de l'hepatite B.
1 - Prelever 10 microlitres de serum et les déposer au fond de l'entonnoir sur le filtre
2 - Pipetter 100 µl de la solution d'anticorps et les déposer dans l'entonnoir. Ils vont être absorbés doucement,en quelques secondes

Après 10 secondes,retourner le dispositif :on voit le filtre s'impregner des fluides qui arrivent puis se mouiller completement.Si un antigène HBs etait present dans ce serum,une coloration bleue se serait produite en quelques secondes après l'impregnation totale du filtre.

Un serum etalonné de l'Institut national de la Transfusion Sanguine de Paris,contenant 1000 nanogrammes d'antigène HBs, a été testé puis dilué 10 fois,puis 100 fois,1000 fois, ... jusqu 'à 1 Milliard de fois (10 puiss 9) soit un millipicogramme/ml dans du serum negatif verifié en PCR

Les resultats suivants ont été observés de façon repetitive :

| | | | | | | | | | |
|---|---|---|---|---|---|---|---|---|---|
| Log de dilution | 1 | 3 | 4 | 5 | 6 | 7 | 8 | 9 | 10 |
| Intensité coloration | +++ | +++ | +++ | +++ | ++ | ++ | ++ | + | 0 |

Le dispositif utilisé ne contenait pas le réactif R4 bipolaire biotine-avidine reputé augmenter la sensibilité.Un essai est donc refait avec un dispositif ultra-sensible et une comparaison est faite avec la technique PCR.

Les dilutions se font à nouveau mais plus loin; jusqu'à 10 puissance 12 (1 micropicogramme/ml ou femogramme/ml).Chaque tube de dilution est divisé en deux tubes. L'un est adressé à la PCR et l'autre est testé avec la version ultra-sensible de ce dispositif.

### Resultats obtenus :

| | | | | | | | | | |
|---|---|---|---|---|---|---|---|---|---|
| Log dilution | 1 | 3 | 5 | 7 | 9 | 10 | 11 | 12 | 13 |
| Intensité de coloration | +++ | +++ | +++ | +++ | +++ | +++ | ++ | 0 | 0 |
| PCR | positif | Positif | Positif | Positif | Neg | Neg | Neg | Neg | Neg |

La PCR n'a rien detecté à partir du tube n°9 contenant 1 millipicogr/ml

Le dispositif present s'est fortement coloré jusqu'au facteur de dilution 11 avant de s'eteindre totalement et brusquement : explication: en appliquant la règle des molarités/nombre d'Avogadro et en partant du tube initial qui contenait 1000 ng/ml d'antigène, sachant que le poids moleculaire de l'antigène HBs est de 50.000 daltons , il ne restait plus suffisemment de molecules d'HBs (20 molecules par millilitre) dans la dilution de facteur 11 pour avoir au moins une molecule dans les quelques microlitres deposés dans le dispositif.La forte coloration de la dilution 10 et son interruption brutale en dilution 11 confirme bien qu'un ruban de plusieurs dizaines de réactifs R4 s'est formé à la suite de l'anticorps qui a detecté quelques molecules d'HBs.

### DETECTION D'ANTICORPS SERIQUES

### Exemple : Detection d'anticorps anti p24 du virus HIV

Mode operatoire : des molecules p24 de virus HIV (temoins inactivés de contrôle +) et des anticorps polyclonaux anti p24 sont fournis par Virostat (USA) pour servir à « positiver » serologiquement des serums negatifs moins dangereux à manipuler. Sur les molecules de p24 on applique le protocole decrit plus haut : greffage de SMCC (R1) sur les sites epitopiques antigeniques de la proteine (contenant des ponts disulfures) et d'Avidine (R3) sur un autre pôle non concerné par la liaison aux anticorps.

Sur le filtre sont normalement greffés R2 (SPDP) et plus loin R4 (biotine-peroxydase).Le dispositif ne necessite pas d'amplification du signal car les anticorps seriques circulent très tôt à des doses considerables.

Une gouttelette de serum negatif est deposée sur le filtre puis on verse 100 µl de la solution de p24 preparée : la fenêtre signal se mouille sans se colorer.

Puis on prepare les serums positifs en ajoutant aux serums negatifs des doses decroissantes d'anticorps polyclonal anti p-24

Le dispositif se colore même aux dilutions très avancées.

### DETECTION DE BRINS D'ADN

Principe : une sonde nucleique capable de reconnaître un brin d'ADN correspondant est preparée à l'avance sur le même principe que les anticorps : greffage de R1 sur les sites de liaisons au brin recherché,et greffage de R3 en bout de sonde.Sur le filtre,R2 est un réactif qui se lie à R1 et fixe l'ensemble R3-R1-sonde qui n'a pas rencontré le brin recherché.Plus en aval,l'ensemble R4 + traceur R4 sera entrainé par R3 si la sonde qui s'est couplé au brin correspondant trouvé dans l'échantillon,a echappé à R2. L'option R4 avidine biotine,source d'extrême sensibilité, trouve ici tout son intêret car les brins d'ADN recherchés sont parfois au nombre de quelques brins par test (prelevements de cheveux,d'empreintes,de traces de sang)

### MULTIDETECTION (SCREENING) à partir d'une SEULE gouttelette d'échantillon

Principe : Plutôt que de rechercher affection par affection,il est possible de proceder à une detection simultanée de nombreux antigènes differents (le total peut atteindre la trentaine en augmentant legerement la taille du dispositif) à partir de la même goutte d'échantillon.

Au lieu d'utiliser et de preparer un seul anticorps,l'on associe dans la même solution d'anticorps préparés,plusieurs anticorps differents.Si le test se colore c'est qu'au moins une des affections est presente.ll convient alors de faire test par test pour determiner laquelle est presente.

Exemple : en centre de transfusion : screening de l'Hepatite A,hepatite B,Hepatite C et HIV (proteine p24)

### DISPOSITIF MULTITEST

Dans une même carte sont insérés plusieurs dispositifs identiques mais chacun devant être utilisé avec un anticorps different.

Exemple : sur une carte de 4 tests identiques on dépose 4 gouttelettes de sang ou de serum.Sur le premier dispositif on depose ensuite une solution d'anticorps anti Hepatite A antigène,sur le dispositif suivant on depose une solution d'anticorps anti HBs, le suivant,un anticorps anti Hepatite C et enfin sur le quatrième,un anticorps anti p24 (HIV)

Si le test du paragraphe precedent s'etait coloré,ce test permettra de dire quelle est l'affection presente.

Ce système est aussi valable pour un groupe de marqueurs tumoraux ou pour un ensemble de bacteries,etc

### DISPOSITIF DE MONITORING QUOTIDIEN

Dans un, dispositif long pouvant contenir jusqu'à 30 tests, il est possible de faire un test par jour ou par semaine.Le medecin qui prescrit un traitement peut être interessé par le resultat (disparition de l'antigène causal) ou par l'apparition d'un effet secondaire detectable (lyse hepatocellulaire, anomalie pharmacogenomique,etc) ou par l'evolution d'un marqueur tumoral au fil du traitement.

### DETECTION PAR BIOPSIE

Le dispositif present (depose du materiel à tester dans l'entonnoir puis au même endroit une solution d'anticorps specifique de la substance recherchée dans la biopsie,absorptoin immediate de cette solution par le filtre et signal quelques secondes après) presente tous les atouts pour completer harmonieusement l'anatomo-pathologie de façon pratique et abordable,permettant des biosies per operatoires à resultat instantané au lieu de laisser attendre le patient anesthesié pendant de longs moments,ou des prelevements locaux (gorge,genitaux,etc) plus faciles.

Exemple choisi : frottis cervical chez une femme presentant des lesions ulcereuses dysplasiques saignotantes du col uterin,examen pratiqué pendant la consultation gynecologique au cabinet du medecin qui pose quatre hypotheses diagnostiques possibles : deux MST (Chlamydiae - Syphillis) une mycose (Candida albicans) un virus (Papilloma virus).Les traitements respectifs de ces affections sont fondamentalement differents, voire même opposés (un antibiotique peut aggraver une mycose) et le medecin est souvent confronté à une impasse therapeutique dans l'ignorance de l'agent causal.

Materiel : Un ecouvillon sterile et quatre dispositifs separés ou sur la même carte.Quatre solutions d'anticorps differents specifiques de ces agents infectieux. Mode operatoire :

Le prelevement de mucosités cervicales par ecouvillon est deposé par une simple touche au contact du filtre au fond de chacun des quatre entonnoirs avant d'y verser l'anticorps different pour chacun des tests.Le test coloré correspondra au diagnostic.

Cette methode de depose d'échantillons solides avant de verser l'anticorps correspondant,ne se limite pas à l'anatomo-pathologie humaine ;elle s'applique naturellement aux prelevements systematiques vétérinaires (moelle,cerveau,visceres,dans l'ESB),ecologiques (détection d'hydrocarbures,de dioxines) agroalimentaires (salmonnelloses,listeria,rotavirus des gastroenterites,etc ) epidemiques et endemiques (virus Ebola en Afrique,Malaria,fievre jaune, paludisme bilharzioses des pays en voie de développement).Les domaines sont vastes.

### DETECTION PRECOCE DE PATHOLOGIES INFECTIEUSES

La revue Impact Medecin (No 1053 du 5 Mai 1997) classant les 10 affections les plus meurtrières dans le monde, ont placé 7 maladies infectieuses.En terme de nombre de victimes,les infections viennent largement en tête : 21,7 Millions de decès par an (contre 7,2 pour les cardiopathies coronariennes, 6,3 pour les cancers et 4,6 pour les affections cerebrovasculaires)

Mais en dehors de l'aspect infectieux dangereux en lui même,l'univers bacterien interesse fortement depuis quelques années le monde medical. Une relation est aujourd'hui etablie entre de nombreuses affections anciennement considérées comme non-infectieuses et un agent causal bacterien.L'on sait aujourd'hui que l'ulcus gastro-duodenal est dû à la presence dans l'estomac,d'une bacterie : Heliobacter Pylorii.

Des preuves s'accumulent de jour en jour pour impliquer indirectement des agents infectieux dans la pathologie cardio-vasculaire par augmentation sensible de la C Reactive Proteine.Plus precisément,le professeur Ridckler (Boston) et son equipe ont demontré dans une etude cross-sectionnelle portant sur 233 patients (37% femmes et 63% hommes - âge moyen 57 ans) recrutés au NIH et dont 158 (68%) avaient des lesions coronariennes prouvées en angiographie,qu'il y avait une correlation entre la seropositivité pour certains agents pathogènres (Chamydia pneumoniae,CMV, herpes HSV1 et 2 et virus hepatite A) et la presence d'une coronoropathie authentifiée.Seuls 3% des patients n'avaient jamais été infectés par aucun de ces agents alors que 70% l'avaient été à au moins deux agents.Mais le plus intéressant est que la prevalence passe à 85% pour les patients ayant contracté cinq agents pathogènes.Aussi interessant,la CRP suit la même courbe avec 1 mg/ml chez les sujets sains,7. mg/ml pour les porteurs de deux agents et depassant les 10 mg/ml chez ceux infectés par plus de quatre agents.

Le present dispositif permet d'apporter une forte contribution à la lutte contre le fleau infectieux à l'echelle mondiale :
1 - en terme de prevention (examens systematiques plus simples,rapides,très peu coûteux)
2 - en terme de traitement précoce : le médecin qui fait un prélèvement gastrique au cours d'une gastro-fibroscopie peut avoir la reponse immediate de recherche de l'Heliobacter Pylorii ce qui lui permettra de mettre en place aussitôt le traitement devenu spectaculairement efficace de l'ulcus par des antibiotiques alors qu'auparavant il fallait attendre le résultat de l'anatomo-pathologie.

### EXEMPLE : DETECTION DE L'ANTIGENE LPS du CHLAMYDIAE

Un anticorps monoclonal de haute affinité et specifique de l'antigène LPS du Chlamydiae Trachomatis est marqué avec R1 (SMCC) et R3 (avidine)

L'échantillon est un prelèvement vaginal sur ecouvillon sterile immediatement deposé sur le dispositif.Les methodes anterieures necessitaient un traitement special de l'échantillon par un detergent particulier : le Chaps (Protocole de Diagnostics Pasteur - Marne la Coquette - France) puis un vortexage intense et long afin de lyser les cellules infectées et liberer ainsi de grandes quantités de particules bacteriennes de chlamydiae dont l'antigène LPS qui n'aurait pu être dectecté autrement

Dans le cas present le dispositif est suffisemment sensible pour detecter les traces d'antigène LPS dans le prelèvement (eclatement spontané des cellules infectées) sans avoir besoin de lyser toutes les cellules du prelevement.

Après avoir deposé le prelèvement sur le dispositif,on dépose 100 µl de la solution d'anticorps anti-LPS qui est absorbée en quelques secondes.On retourne le dispositif pour lire le résultat : la coloration bleue signifie la présence de Chlamydiae Trachomatis dans le prelèvement.

Cette méthode peut s'appliquer à toutes les MST (Maladies sexuellement transmissibles) mais aussi à la détection du papillomavirus au niveau du col uterin en cas de dysplasie suspecte,ou la détection de germes dans les salpyngites ou autres infections annexielles (tuberculose uro-genitale par exemple)

### DOSAGE HORMONAL :SUIVI DE L'HORMONE HCG (Hormone chorionique gonadotrophique) DANS UN CANCER DE TESICULE

Un homme jeune est suivi et traité pour cette affection qui a été opérée trente jours auparavant (J30).Les résultats du test ont été comparés aux resultats du laboratoire de ville auquel a été confié le patient.

| | | | | | | |
|---|---|---|---|---|---|---|
| Durée du suivi | J30 | J60 | J70 | J80 | J90 | J120 |
| Coloration du test | ++ | ++ | ++ | + | 0 | 0 |
| Resultats laboratoire | 50Ul/ml | 6 Ul/ml | < 1 Ul/ml | <1Ul/ml | <1Ul/ml | <1Ul/ml |

| | | | | | | |
|---|---|---|---|---|---|---|
| (c'est à dire non detectée) | | | | | | |

Pourtant jusqu'à J80 il subsistait de l'HCG.ce qui a permis de laisser encore en place le processus des chimiotherapies et d'eviter des rechutes catastrophiques s'il avait été arrêté sur la base des données du laboratoire de ville.

### CARDIOLOGIE

La détection de la troponine I serique libérée par les cellules myocardiques necrosées au decours d'un infarctus aigu a fait l'objet d'une publication len 1995 dans le JAMA ; 273 ; 1279-1282 par Elliott M. Altmann et David B.Sacks : Evaluation of a rapid bedside assay of detection of serum cardiac Troponin T

Le système utilisé etait basé sur la migration sur une bande,d'anticorps marqués aux sels d'or qui s'agglutinent sur une ligne pourpre plus ou moins visible selon le taux de troponine T detectée.Cette ligne n'apparaît (faiblement visible) qu'à partir de 0,2 ng/ml et en 20 minutes.Ce qui fait que la detection n'a pu se faire dans les toutes premières heures : le tableau suivant montre la faible sensibilité de cette methode (33% les 2 premieres heures, 50% entre 2 et 4 heures,75 % entre 4 et 8 heures et 86% au delà de 8 heures.

Etant donné l'extrême sensibilité du système objet de la presente invention,la détection aurait été faite dans les premières minutes comme le prouve l'essai suivant pratiqué avec des doses infinitesimales de troponine TnTc (troponine provenant du myocarde à la difference de la troponine dEs muscles squelettiques),en comparaison du test rapide d'Elliott Altmann portant sur 100 personnes admises en urgences cardiologiques pour syndrome infarctus.

Le système objet de la presente invention a utilisé des anticorps hautement specifiques de la TnTc ne croisant pas avec la troponine squelettique,ainsi que de la Troponine TnTc purifiée pour etalonner des serums negatifs.L'ensemble anticorps et troponine TnTc a et fourni par le laboratoire Boerhinger.Les marquages n'ont pas été maxiamaux (R1 : SMCC R2 : SPDP R3 Avidine R4 : Biotine peroxydase non polymerisée (sans avidine))Le dispositif est simple : 26X10 mm et Signal Glucose oxydase DG TMB.

### Resultats

| | | | | | | |
|---|---|---|---|---|---|---|
| Temps ecoulé | 0-15 mn | 16-30 mn | 31-60mn | 1-4 H | 4-8H | > 8 H |
| Nombre cas | 5 | 7 | 13 | 23 | 10 | 42 |
| Detectés par Elliott Altman | 0 | 1 | 7 | 18 | 8 | 36 |
| | TnTc < 0,2 ng/ml | | TnTc > 0,2 ng/ml | | | |

### Comparaison avec le système présent :

| | | | | | |
|---|---|---|---|---|---|
| Serums negatifs positivés à : | 0,01 picog/ml | 0,1 picog/ml | 1 picog/ml | 10 picog/ml | 1 nanogr/ml |
| Nombre tubes | 10 | 10 | 10 | 10 | 10 |
| Resultats positifs | 10 | 10 | 10 | 10 | 10 |

La troponine a été choisie pour illustrer la sensibilité de cette technique mais surtout pour son excellente specificité.Les autres enzymes cardiaques sont peu spécifiques : CKMB massive, CK, LDH, ASAT.

### CANCEROLOGIE

Un chapitre important sera consacré à ce domaine plus loin dans la partie genomique et proteomique dans laquelle sera decrite l'implication du present système dans la relation entre gènes,produits de gènes translatés ainsi que l'accompagnement possible des puces à ADN.

Les marqueurs tumoraux classiques decouverts à partir de 1990 gardent encore un certain interêt diagnostique (calcitonine dans le cancer medullaire de la thyroïde, hCG dans les tumeurs placentaires ou testiculaires) ou de suivi de l'affection au cours du traitement (CA 15-3 dans le cancer du sein,CA125 dans celui de l'ovaire,PSA pour la prostate,AFP et ACE pour le foie,CA 19-9 pour le pancreas,NSE pour le poumon,etc)

Tout récemment des equipes internationales ont demontré le rôle du virus d'Epstein Barr dans de nombreux cancers et le rôle de la telomerase qui favorise la cancerisation de cellules porteuses d'oncogènes (l'inhibition de la telomerase entraine la mort de cellules cancereuses - Dr Robert Weinberg « Nature Medicine,Octobre 99 pp 1164 et 1129)

Le dispositif et procédé objets de la presente invention peuvent tout à fait detecter des doses infinitesimales de telomersase ou de son excès,des traces de virus Einstein Barr et d'une manière generale tous les oncogènes internes et externes au corps humain susceptibles de canceriser la cellule normale.(oncogènes mutés tels RAS ou p53,proteines virales comme le papillomavirus)

Il est possible d'utiliser un dispositif simple pour suivre un marqueur mais egalement l'option multitest ou multidetection (plusieurs tests differents sur la même carte ou plusieurs marqueurs à partir de la même goutte d'échantillon).L'option hypersensible ne s'impose guère ici puisqu'au contraire c'est du semi-quantitatif pur (ces marqueurs existent chez les sujets sains et c'est leur excès qui traduit l'etat pathologique).

Par contre,dans le chapitre consacré plus loin à la cancerologie en genomique-proteomique,la sensibilité sera l'atout de ce système puisque les produits de translation genique par exemple,au debut d'une afffection maligne,sont actuellement indetectables par les methodes courantes.De plus,l'analyse de microéchantillons par les puces à ADN detecte des molecules d'ADN translaté recherché et le present système accompagnera harmonieusement la puce à ADN pour la detection de protéine cancerigène correspondante est même permis comme on le verra plus loin d'imaginer un système dans lequel la même goutte sera lue par la puce à ADN avant d'être absorbée par le dispositif present preparé pour detecter jusqu'à 30 proteines dont l'ADN est recherché.

### DETECTION DE RECEPTEURS MEMBRANAIRES ET DES CYTOKINES

Principe : de nombreux ligands circulants extracellulaires se lient à leur recepteur propre sur la membrane des cellules.Cette union produit l'emission d'un signal tel que la polarisation d'un radical Tyrosine dans la partie intracellulaire du recepteur,ou l'activation d'une protéine G et la transformation d'AMPcyclique en ATP ainsi que bien d'autres reactions intracellulaires.Le principe est donc qu'au lieu d'utiliser des anticorps pour detecter le recepteur,on utilise son ligand preparé selon la même methode que les anticorps (R1 sur les sites de liaisons, R3 plus loin, R2 et R4 sur le filtre)

Le present système permet donc le dosage de tout le reseau des cytokines (Interleukines,facteurs de croissance,hormones telles oestrogènes (cancer du sein),ou l'insuline qui ont des recepteurs bien indentifiés.Ce dosage apporte souvent une indication de pronostic et guide 1 etraitement.

Il en est de même des recepteurs membranaires dont l'importance est à present demontrée dans la reconnaissance de proteines dangereuses (virus,proteines tumorales,etc),induisant ainsi les defenses des cellules qui supportent ces récepteurs.

En cancerologie,un intéressant travail a été effectué par une equipe de l'Université de Californie sur les lymphocytes NK (Natural Killers) ou d'autres cellules immunitaires comme les CD8 tous deux porteurs du recepteur membranaire NKG2D.

Dès qu'une cellule devient cancereuse elle produit des proteines tumorales reconnues par le recepteur NKG2D qui declenche les defenses lymphocytaires : anticorps detruisant les cellules tumorales. (Adelheid Cervanka et Lewis Lanier - Revue Immunity - Juin 2000)

Malheureusement,chez certaines personnes ces defenses ne se mettent pas en place (deficit immunitaire,etc) et le cancer se developpe. L'interêt de ce travail est qu'un vaccin est possible en vaccinant les sujets à risque contre les proteines des cellules tumorales : ces dernières etant connues,identifiées.Malheureusement encore une fois,dans les cancers sevères,ces proteines tumorales ne sont pas exprimées et donc les lymphocytes NK n'ont pas de prise sur les cellules tumorales : il semble que ces cancers sevères reduisent au silence leurs propres gènes codant pour ces proteines (comme par un incroyable reflexe de camouflage contre les lymphocytes tueurs NK).Un des objectifs est donc de « reveiller » ces gènes de cellules cancereuses codant pour les proteines tumorales et ainsi permettre aux defenses immunitaites Ly NK et CD8 d'intervenir.

La presente invention se propose donc de definir la situation immunologique du patient en detectant la presence ou l'absence de proteines tumorales tant sur un plan diagnostic que de suivi therapeutique.

### DETECTION DE PATHOLOGIES PLUS RECENTES

### Exemple 1: DETECTION DU PrP (PRION PATHOLOGIQUE) dans un homogeneisat de cerveau bovin

Le matériel est gracieusement fourni par l'equipe de Monsieur Jacques Grassi du CEA (Commissariat à l'Energie de Saclay - France) directeur du Laboratoire de biologie moleculaire des proteines.

Materiel fourni :
1 - Un anticorps monoclonal : SF33 specifique du PrP recombinant
2 - Un tube de solution PrP recombinante à 1 mg/ml
3 - Un flacon d'homogeneïsat de cerveau bovin normal testé negatif en PCR et traité par detergents et urée 3 M.

Le but de la manipulation est de demontrer l'extrême sensibilité de la methode qui manque aux methodes classiques laissant echapper des animaux très faiblement ou recemment atteints mais neanmoins dangereux à la consommation.La comparaison est faite par rapport à trois autres methodes : l'une classique : Elisa,la seconde : PCR classique et la troisième : ImmunoPCR mise au point par une equipe japonaise et utilisée par l'equipe du Professeur Hochstrasser à l'hopitall cantonnal de Genève-Suisse

Les mesures,pour être coherentes sur le plan international sont en Moles de PrP par litre d'homogenïsat.

Cependant : il convient de preciser qu'il s'agit de detection de PrP recombinante diluée dans un homogeneïsat de cerveau normal et qu'il ne s'agit donc pas des conditions normales de cerveaux pathologiques « d'abattoir ».En effet,le prion existe sous deux formes :la forme classique (2 helices dans la structure quaternaire de la molecule) et la forme pathologique (1 helice).Les anticorps monoclonaux les plus perfectionnés ne sont pas parvenus à faire la difference : ils les detectent indifferemment tous les deux.Une equipe europeenne a reussi à detruire le prion normal par un traitement puissant de l'homogeneïsat de cerveau à tester,par des detergents et Urée 3M.Le prion pathologique PrP resiste à ce traitement et est donc detecté par les anticorps monoclonaux.

Mode opératoire : Après vortex intense, on dépose sur un dispositif de 20 mmX10 mm 10 µl de l'homogeneisat de cerveau normal.Puis 100 µl de solution d'anticorps monoclonal preparé avec R1 et R3.Au bout de quelques secondes la fenêtre du resultat est imbibée de fluides et ne se colore pas.

On dispose 8 tubes sur un portoir,contenant chacun 100 µl d'homogeneïsat de cerveau normal numerotés de 1 à 8

La solution de PrP recombinant à 1 mg/ml (soit 10 puiss-3 g / PM 10.000 =10 puiss - 7 Moles/ml ou 10 puiss -10 Moles/L) est diluée 100 fois dans le tube 1 (1 µl de PrP dans les 100 µl d'homogeneïsat normal) puis de tube en tube pour avoir les molarités selon le tableau suivant:

| Logarithme des molarités/litre | | | | | | | | |
|---|---|---|---|---|---|---|---|---|
| Méthode | -12 | -15 | -16 | -17 | -18 | -19 | -20 | -21M/l |
| | | | | | | | | |
| Elisa | ++ | 0 | 0 | 0 | 0 | 0 | 0 | 0 |
| PCR | Positif | Positif | 0 | 0 | 0 | 0 | 0 | 0 |
| ImmunoPCR | Positif | Positif | positif | 0 | 0 | 0 | 0 | 0 |
| Dispositif Present | +++ | +++ | +++ | ++ | ++ | + | 0 | 0 |

### Exemple 2 : LA MALADIE D'ALZHEIMER

Deux arguments biologiques viennent aujourd'hui conforter le diagnostic clinique de la maladie d'Alzheimer:
1) la presence serique à un taux très faible d'apolipoproteine E4 due à la presence de 1 ou 2 allèles e4 du gène de l'apolipoproteine sur le chromosome 21 (alors que les porteurs d'allèles e2 ou e3 ne font jamais cette affection).(Gertrude H - Sergiewsxy center,NY-USA dans New england Journal of Medicine, 19 Fevrier 1998, p 506)
2) la présence intracerebrale de la proteine pathologique APP (Amyloïd Precursor Protein) responsable des depôts encephaliques de proteine Beta-amyloïde caracteristique de la maladie.La production de cette proteine anormale est dûe à une mutation du gène APP (substitution de ILE pour VAL) sur le chromosome 21.De nombreuses publications prouvent la presence à des doses indetectables de proteine beta-amyloïde dans le sang ou même dans les tissus extra-cerebraux (un test de detection de cette proteine dans la biopsie d'un fragment dermique de l'avant-bras a été tout recemment presenté).

L'allèle e4 offre une sensibilité de 70% (3 malades sur 10 n'avaient pas l'allèle e4 mais plutôt e2 ou e3) et une specificité de 70% (3 personnes saines sur 10 avaient l'allèle e4).Par contre le gène APP est present chez tous les malades (« Nature » du 21 Fevrier 1991, vol 349,n° 6311 p 704-706 - Dr M-C Chartier-Harlin depart.Biochimie - St Mary's Hospital Medical School - London)

### PROTOCOLE :

1 - selection de dix patients porteurs de la maladie d'Alzheimer sur des bases cliniques sûres et de dix sujets du même âge sains
2 - phenotypage du gene apoE et du gène APP en etudiant leurs allèles sur le chromosome 21
3 - dosages biologiques classiques de l'apoE4 et de la protéine APP
4 - Dosage par la présente méthode en utilisant les mêmes serums que ceux utilisés par le laboratoire classique,des mêmes patients et au même instant.Les anticorps utilisés ont été fournis par Sigma Neurosciences

Research (filiale Sigma-Israel à Kiryat Weizmann).Le dispositif retenu mesure 26X10 mm. Etant données les quantités infinitésimales (10 puiss-19 moles/ml),le volume serique a été augmenté à 10 µl pour rehausser encore la sensibilité et le système d'amplification du signal utilise la chaîne avidine-peroxydase-biotine-avidine-peroxydase-biotine-etc décrite plus haut. Une croix dans le tableau suivant est une coloration visible bleue rapide.

### Mode operatoire :

On dépose une goutte de sérum dans chacun des quatre systèmes installés sur la même carte puis on dépose le contenu de la première solution (anticorps anti-proteine APP) dans le premier système,le contenu de la deuxième solution (anticorps anti apoE2) dans le second système, le contenu de la troisème solution (anti apoE3) dans le troisième système,le contenu de la quatrième solution (anti-apoE4) dans le quatrième système.On retourne la carte après quelques secondes

### Résultats obtenus

| | | | | | | | | | | | |
|---|---|---|---|---|---|---|---|---|---|---|---|
| | Tube: | 1 | 2 | 3 | 4 | 5 | 6 | 7 | 8 | 9 | 10 |

| Temoins sains | | | | | | | | | | | |
|---|---|---|---|---|---|---|---|---|---|---|---|
| Allèle e2 | 4 | - | - | - | + | - | - | + | - | - | - |
| Allèle e3 | 3 | - | + | - | - | - | - | - | - | - | - |
| Allèle e4 | 3 | + | - | + | - | + | + | - | + | + | + |
| Gène APP | 0 | + | + | + | + | + | + | + | + | + | + |

| Dosage classique de la proteine | | | | | | | | | | | |
|---|---|---|---|---|---|---|---|---|---|---|---|
| ApoE 2 | 4 | - | - | - | + | - | - | + | - | - | - |
| ApoE 3 | 3 | - | + | - | - | - | - | - | - | - | - |
| ApoE 4 | 3 | + | - | + | - | - | + | - | + | - | + |
| APP | 0 | + | + | + | + | - | - | + | - | + | + |

| Dosage par la presente methode | | | | | | | | | | | |
|---|---|---|---|---|---|---|---|---|---|---|---|
| ApoE 2 | 4 | - | - | - | + | - | - | + | - | - | - |
| ApoE 3 | 3 | - | + | - | - | - | - | - | - | - | - |
| ApoE 4 | 3 | + | - | + | - | + | + | - | + | + | + |
| APP | 0 | + | + | + | + | + | + | + | + | + | + |

Le dosage classique des proteines (Elisa) n'a pas detecté du tout le cas numero 5 (ni protéine apoE4 ni APP,pas l'APP dans les cas numero 6 et 8,pas l'apoE4 dans les cas 7 et 9 (probablement stades biologiques faibles ou precoces)

Le present sytème a retrouvé l'apoE4 chez les 7 malades porteurs de l'allèle e4,l'APP chez tous les malades

**TABLEAU RECAPITULATIF COMPARATIF sur dix malades confirmés cliniquement**

| | SENSIBILITE | SPECIFICITE |
|---|---|---|
| Phenotypage e4 | 70% | 70% |
| Biol.classique apoE4 | 5 sur 7 porteurs e4 | 3 sains negatifs/3 |
| Système present | 7 sur 7 | 3/3 |
| | | |
| Phenotypage APP | 100% | 100% |
| Bil.classique APP | 70% | 100 % |
| Système present | 100% | 100% |

Conclusion : Une détection fine,fiable à 100% en sensibilité et spécificité est possible dans la maladie d'Alzheimer avec le present système à partir d'une gouttelette de serum et probablement de sang total,plasma,salive,LCR ou tout fluide biologique ainsi qu'à partir d'une biopsie même microscopique du derme ou d'un organe en peroperatoire.

La detection repose avec certitude sur la presence concomittante d'APP et d'apoE4. La presence d'APP seule demande une confirmation par phenotypage e4 :si le gène est present,la certitude du diagnostic est acquise.L'absence totale d'APP ET d'apoE4 permet d'exclure,du moins provisoirement,une maladie d'Alzheimer et d'orienter les recherches vers d'autres causes (vasculaires, metaboliques,etc.En resumé :

| Detection | APP | apoE2 | apoE3 | apoE4 | = |
|---|---|---|---|---|---|
| | + | - | - | + | Mal.Alzheimer |
| | + | + | - | - | Mal.Alzh.atypique |
| | + | - | + | - | Id |
| | - | - | - | + | Sujet à risque |
| | - | + | + | - | Negatlf sûr |

### Exemple 3 : SCLEROSE EN PLAQUES (SEP)

C'est le français Hervé Perron (Directeur de l'Unité mixte CNRS-BioMerieux : UMR103 Lyon) qui a decouvert le retrovirus MSRV (Multiple Sclerosis Retro Virus) impliqué dans la Sclerose en plaques (Procced.Nat.Acad. of Sciences Juillet 97,vol 94 p 7583).Il s'agit d'un oncovirus de type C,retrouvé chez des patients atteints de SEP et à present identifié et sequencé.Comme tous les retrovirus,il s'insère dans l'ADN des monocytes du liquide cephalorachidien de patients en phase active et transmet la sequence specifique MSRV pol à l'ARN cellulaire.Sa phylogenie le rapproche des retrovirus MuLV,MMTV, JSRV responsables chrez l'animal,de virions infectieux causant atteintes neurologiques,tumeurs solides, leucemies. Ces virions expriment des superantigènes endogènes circulant à des taux indetectables actuellement .

H. Perron et son equipe ont travaillé sur des cultures de cellules du plexus choroïde de temoins sains et de patients,etude de l'activité retrovirale, clonage et sequençage, hybridation PCR,extraction de l'ARN, synthèse d'ADN et amplification PCR à partir d'échantillons plasmatiques: il a clairement etabli que le virus MSRV produit une proteine puissamment gliotoxique à partir de la cellule infectée,et responsable des lesions gliales cerebrales.

La presence de materiel genetique de MSRV mais surtout de cette proteine dans le sang circulant a été prouvée mais à des taux indetectables par les methodes classiques,ce qui permet le diagnostic par le dispositif objet de la presente invention mais surtout la detection de survenues de crises « actives » de la maladie ainsi que le suivi du traitement.

Cet exemple illustre les possibilités potentielles de detection dans les autres affections retrovirales (suivi des traitements du HIV,etc)

### GENOMIQUE ET PROTEOMIQUE

Le sequençage du genome humain fin Juin 2000 a permis de mieux cerner la proteomique (etude des proteines correspondant à une sequence genetique,leur classement fonctionnel,leur structure,etc).La pharmacogenomique identifie les gènes impliqués dans l'efficacité d'un produit ou au contraire ses effets indesirables.Or l'evaluation de ces données est techniquement impossible aujourd'hui : les proteomes recherchés circulent dans la gouttelette d'échantillon (dans une puce à ADN par exmple) à des doses infrabiologiques indetectables.Les methodes PCR sont très coûteuses et très longues.Quand on sait qu'une hybridation de deux sequences d'ADN prend plusieurs heures et que des milliards de sequences figurent dans le genome,on imagine le coût et les delais pour progresser dans la voie de la genomique.

La presente invention se propose donc d'apporter son extrême rapidité et sensibilité pour accompagner les progrès de la biologie moleculaire et en particulier de la genomique sous tous ses aspects.

### EXEMPLE D'UN TEST DE DETECTION DE PROTEOMES DANS LES OGM (Organismes genetiquement modifiés)

Pour lutter contre les substances toxiques pour les plantes et introduites par des virus,bacteries,champignons ou insectes,des résistances se créent au niveau des plantes elles mêmes qui produisent une molecule anti-toxine (proteome) à partir de leur patrimoine genetique propre.

Les manipulations genetiques sont parvenues à inserer des sequences d'ADN, des gènes,induisant la production de telles substances anti-toxines pour proteger les recoltes.

Tacke E.,Salamini F et Rohde W (1996) dans Nat.Biotechnol. 14 :1597-1601 sont parvenus à inserer un gène de protection contre le virus de la pomme de terre.

Il existe dans la nature un grand reservoir de producteurs de proteines toxiques pour les parasites : le seul Bacillus thuringiensis contient une centaine de gènes codant pour ces proteines et certains de ces gènes ont été greffés dans l'ADN de graines pour les defendre contre ces parasites.(Estruch et Al. 1997 - Nat. Biotechnol. 15 : 137-141)

L'exemple etudié concerne donc la detection de la proteine rP234 du B.Thuringiensis dans une pomme de terre dont la graine a été manipulée genetiquement pour produire cette proteine.

Matériel : une pomme de terre non manipulée (NM) et une pomme de terre manipulée genetiquement (M).Un flacon de contrôle negatif et un flacon positif ainsi que l'anticorps polyclonal correspondant(Adgen - Scotland - GB)

### Mode operatoire :

Des fragments de pomme de terre sont ecrasés et placés dans l'entonnoir avant la dépose d'une solution d'anticorps preparé.La coloration bleue confirme la presence de la protéine rP234.Un test similaire a été pratiqué avec les temoins fournis par Adgen et ont donné le même résultat.

Etude de la sensibilité : les fragments de pomme de terre sont vortexés energiquement dans 1 ml de NaCl 0,9%.Les dilutions du tube positif dans le negatif se font au 1/10 puis au 1/1000,etc La detection a eu lieu jusqu'à la dilution de logarithme 11 avant de disparaître

### GENOMIQUE ET CANCEROLOGIE

### PROTEINE p53

Le gène p53 joue un rôle fondamental regulateur comme suppresseur de tumeur et oriente la cellule vers la reparation ou la mort programmée (apoptose).Dans 50% des cancers,il est muté (sein,colon,poumon notemment) et la cellule ne reconnaît pas ses lesions, il n'y a ni reparation ni apoptose,le genome devient de plus en plus instable rendant la cellule cancereuses puis metastatique.A un stade avancé le gène muté p53 entraîne l'accumulation de son produit et des anticorps anti p53 sont facilement detectables dans le serum.

La presente invention s'est proposé d'apporter son extrême sensibilité à la detection ultra precoce de proteine p53 mutante circulante.Madame le Professeur Varda Rotter de l'Institut de Biologie Moleculaire de l'Institut Weizmann,decouvreur de la p53,a bien voulu fournir des serums de lapins immunisés par une p53 mutante synthetique.Une fois le serum purifié et donc des anticorps isolés puis marqués,des serums de donneurs sains ont été testés comme temoins negatifs.Les temoins positifs par addition de quantités decroissantes de p53 mut Recombinante ont été testés : un signal colorimetrique a été encore visible pour quelques femogrammes de cette proteine p 53 mutante recombinante.Ce qui laisse envisager une validation clinique pour detecter très precocément certains cancers. D'autres gènes jouent un rôle : MSH2 , MLH1 ou encore le gene bax qui n'induit plus l'apoptose qand il est muté.

### LE GENE BRCA1 DANS LE CANCER DU SEIN

Les mutations de ce gène sont responsables de la moitié des cancers hereditaires du cancer du sein et confèrent un risque accru des cancers de l'ovaire et de la prostate. La specificité de l'atteinte de ces organes est dûe à la presence d'oestrogènes (responsables de division cellulaire) et de leurs recepteurs. Le gène BRCA1 normal,inhibe l'activité du recepteur alpha pour les oestrogènes et inhibe donc la proliferation cellulaire en cas de stimulation oestrogenique.La mutation de BRCA1 le rend inoperant sur les récepteurs des tissus sensibles aux oestrogènes et la cellule prolifère.

Le procédé objet de la presente invention,peut combiner la detection de p53 et de BRCA1 mutés circulant à des doses infinitesimales.

### LA BETA-CATENINNE

Des chercheurs israeliens (Pro.Natl Acad Sci, 11Mai 1999, p 5222) ont decouvert un mecanisme moleculaire intervenant dans le developpement de cancer colorectal : un regulateur des facteurs de transcription (la beta-catenine) en s'accumulant dans la cellule,active trop la transcription de la cycline D1 agent de proliferation cellulaire.La beta-catenine est connue pour son rôle structurel dans les jonctions intercellulaires en liant le squelette d'actine aux cadherines des recepteurs transmembranaires d'adhesion inter-cellulaire.Mais elle peut aller dans le noyau former des complexes avec les facteurs de transcription de la famille LEF-1 (Lymphoïd Enhancing Factor) pour y reguler l'expression de gènes specifiques. La beta-catenine est ensuite degradée par un complexe formé de la molecule Tumor-suppressor APC (Adenomatous polyposis coli),de l'axine et du GSK-3 Beta (glycogène synthase kinase)

Soit par mutation du gène de la betacathenine (la nouvelle betacathenine n'est plus reconnue par le complexe qui la degrade en temps normal) soit par mutation du gène de ce complexe (surtout l'APC),une accumulation de beta-cathenine se produit dans la cellule (ceci a été constaté également dans le melanome malin)

La cycline D1, regulateur majeur de la phase proliferative du cycle cellulaire,est anormalement elevée dans 30% des cas de cancer du colon mais son gène,paradoxalement est tout à fait normal,n'est pas muté alors qu'en règle generale,tous les oncogènes sont mutés.Son gène est en fait regulé par un promoteur contenant un site de fixation LEF-1.On a vu plus haut que la betacathenine se liait dans le noyau à un site LEF-1.L'accumulation de beta cathenine (pour cause de mutation ou par mutation des complexes qui la degradent en temps normal) provoque une stimulation permanente du site LEF-1 et donc la promotion continue du gène de la cycline D1 avec proliferation cellulaire.L'equipe israelienne a montré que la mutation « coupable » n'a pas besoin d'apparaître dans la protéine responsable mais dans d'autres molecules qui l'affectent indirectement.

L'intérêt est immense sur le plan thérapeutique : il consiste à cibler les deux mécanismes :
1 - Restaurer la degradation de la betacathenine : si la mutation genique se fait sur le gène APC, l'introduction de gène APC normal,degradant la betacathenine,inhibe l'activation de la cycline D1.
2 - Amplifier le piegeage naturel de la betacathenine par des cadherines cytoplasmiques pour la detourner et l'empêcher d'aller stimuler les promoteurs nucleaires LEF-1 de la cycline D1

Mais l'interêt diagnostique est aussi important : la betacathenine et la cycline D1 ne circulent pas à l'exterieur de cellules normales. Les quantités sont à l'echelle moleculaire et sont vite dégradées par le complexe decrit plus haut (APC,etc).Lorsque la cellule subit la mutation du gène de la betacathenine ou des complexes de degradation,il y a accumulation intracellulaire mais certainement extracellulaire de betacathenine et cycline D1,neanmoins indetectables par des methodes classiques ou complexes,très coûteuses et très longues.

La presente invention propose donc son extrême sensibilité et sa simplicité,pour la detection precoce du cancer du colon par un système unique utilisant les anticorps anti beta cathenine et anti cycline D1. Le système comprendra bien entendu une amplification importante du signal. Il sera particulièrement utile pour un depistage de masse en remplacement des recherches de sang dans les selles,peu fiables.

La présence d'au moins un des deux doit déclencher des explorations poussées (recherche du gene muté pour confirmation et surtout pour introduire une therapie genique avec des gènes reparés,fibro-coloscopie,recherche de sang dans les selles,enquête genetique familiale,etc)

### COUPLAGE PUCES ADN ET DETECTION DE PROTEINES

La decouverte de produits directs de translation ou mutation genique (p53 mutée par exemple) ou de produits indirects (la cycline D1 ou la betacathenine si les gènes APC sont mutés) ne permettra d'affirmer la presence d'un cancer du colon que si la confirmation est donnée par la decouverte de gènes mutés.

L'idée consiste donc à proposer un appareillage (Planche 5 Figure 1) qui associerait les deux techniques dans le même dispositif : le système objet de la presente invention sera inséré (1) dans l'appareillage à côté de la puce ADN (2) .Une goutte d'échantillon sera deposée sur la puce ADN et une goutte sur le dispositif présent. La reponse negative (absence de proteines anormales) evite d'utiliser en vain une puce ADN dont la lecture des résultats est fastidieuse.Un reponse positive exigera alors la confirmation par la puce d'ADN

Mais inversement,la decouverte d'une nouvelle mutation inconnue d'ADN à l'aide d'une puce ADN,entrainera la recherche de la protéine mutée à l'aide du present dispositif,en faisant un screening parmi toutes les molecules suspectées.La decouverte de cette proteine entrainera alors la production de la therapie adequate mais surtout la surveillance de l'evolution de la maladie,la detection de rechutes,etc

### LA PHARMACOGENOMIQUE

Est la suite logique de ce qui precède : une fois etablie la cause exacte de l'affection de la cellule,des traitements vont cibler cette cause mais ils peuvent aussi provoquer des effets secondaires ou être inefficaces.

Le present procédé apportera sa contribution en detectant la production,par ces traitements, de proteines anormales et dangereuses en signalant ainsi qu'une altération genique s'est produite par les dits traitements.La puce ADN viendra apporter la confirmation.

Inversement la decouverte systematique d'anomalies ADN par la puce,entrainera la recherche des proteines anormales qui en résulteraient. Associé aux puces ADN et au decollage de la proteomique-genomique,l'apport de la presente invention est immense en termes de :
- rapidité d'exécution : quasi-instantanée
- precocité de la detection : dès les premières molecules
- survie des patients dans de meilleures conditions qu'actuellement
- suivi therapeutique : ajustement precis des choix therapeutiques : bloquer des gènes, detourner une proteine nocive de son parcours malin, la pieger,la detruire,introduire des gènes « reparés »,.. la medecine moderne sait deja faire tout ça mais manque des moyens simples et sensibles pour être guidée.
- detection d'effets secondaires des traitements sur le genome.
- Et enfin participer à la mise en place d'une banque de données mondiale visant à mettre en face de chaque gène,la protéine correspondante,les diverses mutations possibles,les proteines anormales ainsi produites et les moyens pour y remedier.

## Revendications

1. Trousse pour la détection d'un analyte contenu dans un fluide ou un solide, consistant en
i) un dispositif étant emballé; étiqueté, sous vide dans un sachet opaque, étanche, avec une plaque de desséchant,
ii) une dose d'une solution d'un ligand porteur des groupements R1 et R3, **caractérisé en ce que** le ligand est porteur à l'une de ses extrémités d'un réactif R1, non traceur et non pourvu d'un quelconque pouvoir enzymatique et capable d'une interaction avec R2, selon que R1 est complexé ou non à son analyte, respectivement, et à l'autre extrémité, d'un réactif R3 également non-traceur et non-enzymatique,
et
iii) une pipette pour prélever l'échantillon et le déposer ou le mélanger directement à la solution de ligand porteur de R1 et R3,
le dispositif comprenant
a) un filtre poreux (1) sur la longueur duquel ont été fixés ou déposés successivement
i) des réactifs R2 capables de laisser migrer ou de piéger un ligand porteur de R1 et R3, par l'intermédiaire d'une interaction entre R2 et R1, selon que R1 est complexé ou non à son analyte, respectivement et
ii) en aval de la zone précédente des réactifs R4 associés à une ou plusieurs molécules de traceur enzymatique capables de piéger un ligand R1-R3 par l'intermédiaire d'une interaction entre R4 et R3, ces réactifs R4 étant déposés de manière non fixe et
iii) en aval de la zone précédente des composés capables d'interagir avec les traceurs enzymatique liés à R4 de manière à produire des milliers de molécules par seconde d'une substance X1 au niveau de l'extrémité de ce filtre constituant la zone-signal qui indique l'arrivée ou non du complexe ligand et analyte,
b) un élément supérieur (4) perforé d'un trou (5) en amont des réactifs R2 sur le filtre, permettant de déposer l'échantillon sur le filtre avant d'y déposer au même endroit la solution de ligands préparée
c) un entonnoir (7) au dessus de ce trou (5) pour guider la déposition des solutions de ligand,
d) un élément médian d'un matériau rigide perforé d'un rectangle de la taille du filtre et destine à éviter le gondolement des bords des éléments supérieurs et inférieurs quand ils enserrent le filtre,
e) un élément inférieur (2) est du même matériau rigide mais perforé d'un trou (3) ou d'une fenêtre carrée au niveau de l'extrémité en aval du filtre, protégée par un mince film transparent pour abriter la zone signal de l'air, à l'endroit où est visible ou mesurable le signal provenant des réactions produites en amont dans le filtre,
le filtre étant enserré entre trois éléments a), b) et c) plus rigides.

2. Trousse selon la revendication 1 **caractérisé en ce qu'**elle est identique quel que soit l'analyte recherché à l'exception de la solution de ligand de ligand porteur de R1 et R3, dont le groupement R1 est spécifique de l'analyte recherché.

3. Trousse selon les revendications 1 à 2 **caractérisée en ce que** le dispositif est associe à un appareillage de lecture et de quantification du signal.

4. Trousse selon les revendications 1 à 2 **caractérisée en ce que** R4 est lié à un traceur peroxydase et **en ce que** les réactifs glucose oxydase, D-Glucose et Tetra methyl benzidine ont été déposés successivement et séparément en aval de R4 sur le filtre du dispositif.

5. Procédé pour la détection dans un échantillon fluide ou solide, d'une substance analyte comprenant les étapes successives:
i) déposer une gouttelette d'échantillon (5) sur le filtre (1) au fond de l'entonnoir (7) d'un dispositif selon la revendication 1,
ii) déposer une solution d'un ligand porteur des groupements R1 et R3 au même endroit, **caractérisé en ce que** le ligand est porteur à l'une de ses extrémités d'un réactif R1, non traceur et non pourvu d'un quelconque pouvoir enzymatique et capable d'une interaction avec R2, selon que R1 est complexé ou non à son analyte, respectivement, et à l'autre extrémité, d'un réactif R3 également non-traceur et non-enzymatique,
iii) incubation dudit dispositif de manière à laisser migrer le mélange ainsi formé en aval du filtre
afin qu'en l'absence d'un analyte, R1 se lie à un réactif R2 fixé sur le filtre dudit dispositif, immobilisant ainsi le ligand sur ce filtre,
et afin qu'en présence d'analyte, le réactif R1 du ligand est occulté et neutralisé, laissant ainsi migrer le complexe d'analyte et ligand, qui se lie alors en aval dans le filtre par l'intermédiaire du réactif R3 avec un réactif R4 non fixé sur le filtre, qui est associé à une ou plusieurs molécules de traceur enzymatique,
iii) détection dans une zone-signal en bout du filtre des milliers de molécules par seconde d'une substance X1 produits par l'intermédiaire de la moitié traceur enzymatique du complexe entre analyte, ligand R1-R3 et R4 lié à une ou plusieurs molécules de traceur enzymatique.

6. Procédé selon la revendication 5, **caractérisé en ce que** la zone signal est en fait l'extrémité du filtre sur lequel une solution de substrat chromogène a été déposée pour réagir avec le composé x1.

7. Procédé selon la revendication. 6, caractérisa en ce que le composé x1 est produit par l'intermédiaire d'un traceur peroxydase lié à R.4 et par les réactifs a) glucose oxydase et b) D-Glucose, **caractérisé en ce que** le composé x1 réagit avec le réactif c) Tetra methyl benzidine, et **caractérisé en ce que** les réactifs a), b) et c) ont été déposés successivement et séparément en aval de R4 sur le filtre du dispositif.

8. Procédé selon la revendication 5 **caractérisé en ce que** le procédé comprend une étape préalable au cours de laquelle l'analyte est mis en contact avec une certaine quantité de ligands ne comprenant ni R1 ni R3, correspondant au taux d'analyte normal, dans le but de neutraliser une certaine quantité d'analyte ainsi formant la gouttelette d'échantillon de l'étape i) de la revendication 5, ce qui permet des dosages semi-quantitatifs, c'est à dire correspondant à un signal d'excès de l'analyte par rapport à la normale.

9. Procédé selon l'une quelconque des revendications 5 à 8 **caractérisé en ce que** l'analyte est un antigène et le ligand un anticorps spécifique de cet antigène, ou inversement **en ce que** l'analyte est un anticorps et le ligand un antigène correspondant.

10. Procédé selon les revendications 5 à 8 **caractérisé en ce que** l'analyte peut être toute molécule capable de reconnaître et se lier à une ligand partenaire correspondant R 1.

11. Procédé selon la revendication 10 **caractérisé en ce que** l'analyte est un brin d'ADN recherché et le ligand R1 correspond à une sonde moléculaire marquée capable de reconnaître ce brin d'ADN recherché,

12. Procédé selon les revendications 5 à 11 **caractérisé en ce qu'**un grand nombre de ligands différents mais préparés de façon identique à R1 et R3, sont associés dans la même solution qui est déposée à la suite de l'échantillon dans lequel on recherche au mois un parmi des dizaines d'analytes correspondants aux dizaines de ligands associés.

13. Procédé selon la revendication 12 **caractérisé en ce que** si au moins un des analytes est présent dans l'échantillon un signal est fourni, lequel signal est aussi proportionnel au nombre d'analytes différents détectés.

14. Procédé selon les revendications 5 à 6 **caractérisé en ce que** l'amplification importante du signal obtenu permet une sensibilité de détection a l'échelle moléculaire de tous les analytes.

15. Procédé selon la revendication 14 **caractérisé en ce qu'**il permet de détecter des substances produites par des affections malignes dues à une translation ou mutation génique, telles que les cancers, en particulier le cancer du colon, la maladie d'Alzheimer, et un infarctus du myocarde.

## Claims

1. A kit for the detection of an analyte present in a liquid or a solid, consisting of :
a. A device packaged, labelled, under vacuum in an opaque container, hermetically closed and containing a desiccant,
b. A volume of a solution containing a ligand with groups R1 and R2, **characterised by** the ligand carrying at one its extremities a reagent R1, not carrying a label or having any enzymatic action and capable of reacting with R2, wherein R1 is complexed or not to its analyte, and at the other extremity a reagent R3 which is also not a label or having any enzymatic action,
And
c. A pipette which is used to recover the sample and to place or mix directly with the ligand solution carrying reagents R1 and R3
The device is comprised of
1. a porous filter (1) onto which is fixed over the complete length or successively placed
a. reagents R2 capable of respectively migrating or trapping a ligand carrying R1 and R3, through the interaction of R1 and R2, according to whether R1 is complexed or not to its analyte and
b. down stream from the precedent zone reagents R4 associated with one or more than one enzymatic tracer molecules and capable of trapping a ligand R1-R3 through the interaction with R4 and R3, the reagents R4 being placed in a manner where they are not immobilised and
c. down stream from the precedent zone components capable of interacting with the enzymatic tracers coupled to R4 in a manner to produce multiple molecules per second of a substance X1 at the extremity of the filter and which constitutes a signal zone indicating the arrival or not of the complex ligand or analyte.
2. A superior component which is perforated with a hole (5) up stream from the position of R2 on the filter, allowing the placing of a sample on the filter prior to placing on the same position the ligand solution,
3. A funnel (7) place over the hole (5) which guides the placing of the sample and ligand solutions,
4. A sandwiched rigid component perforated with a rectangular hole of the dimensions of the filter which keeps the filter edges from warping when inserted,
5. an inferior component of the same rigid material but perforated with a hole (3) or a square window at the extremity of the down stream portion of the filter, protected by a transparent film over the signal zone, to protect this visible or measurable signal produced by the reactions from the reagents in the filter from air,
The filter is inserted between the rigid components 1, 2 and 3.

2. A kit according to claim 1 which is **characterised** as being identical no matter what analyte is being analysed with the exception of the solution containing the ligand carrying R1 and R3 and where the group R1 is specific for the analyte being analysed

3. A kit according to claim 1 and 2 which is **characterised by** the device being associated with a instrument capable of detecting and quantifying the signal

4. A kit according to claims 1 and 2 **characterised by** reagent R4 is coupled to a peroxidase tracer and where the reagents glucose oxidase, D-glucose and tetra methyl benzidine have been place successively and separately down stream from R4 on the filter component of the device

5. A procedure for the detection of an analyte in a liquid or solid comprising of the following successive steps,
a. Place a drop of sample (5) in the funnel (7) on the filter (1) on the device according to claim 1,
b. Place a drop of the ligand solution containing the specific ligand carrying at its extremities a reagent R1, not a label and without any enzymatic activity and capable of reacting with reagent R2, and where the reagent R1 is complexed or not to its analyte and at the other extremity a reagent R3, not a label or without any enzymatic activity,
c. Incubation of the said device in a manner to allow the sample and ligand mixture to migrate down the filter so that, in absence of analyte, R1 is bound to a reagent R2 fixed on the filter of the said device, thus immobilising the ligand on the filter and in the presence of analyte, the reagent R1 of the ligand is blocked and neutralised, leaving the complexed ligand and analyte to migrate which reacts down stream in the filter by action of reagent R3 with reagent R4 whioch is not fixed in the filter and which is associated with one or more than one enzymatic tracer molecules
d. Detection in the signal zone at the extremity of the filter the generation of multiple molecules per second of a substance X1 produced by the action of the enzymatic tracer of the complex comprising of the analyte, ligand R1-R3 and R4 linked to one or more than one enzymatic tracer molecules.

6. Procedure according to claim 5, **characterised by** the signal zone is positioned at the extremity of the filter on which the chromogen substrate has been placed to react with the component X1.

7. Procedure according to claim 6, **characterised by** the production of component X 1 by the action of a peroxidase label linked to R4 and by the reagents a) glucose oxidase, b) D-glucose and c) tetra methyl benzidine and **characterised by** these reagents being placed successively and separately down stream of R4 on the filter of the device

8. Procedure according to claim 5 **characterised by** the procedure comprising of a step prior to when the sample is in contact with a certain quanity of ligand which does not carry R1 nor R3 and which corresponds to a normal level of analyte, with the aim of neutralising a certain quanity of analyte in the sample drop as **characterised in** step a) of claim 5 and which allows a semi-quanification of the analyte, generating a signal in excess as compared to the normal.

9. Procedure according to claims 5 to 8 **characterised** where the analyte is an antigen and the ligand an antibody specific for the antigen or inversely where the analyte is an antibody and the ligand an specific antigen corresponding to the antibody

10. Procedure according to claims 5 to 8 **characterised by** analytes which can be any molecule capable of binding to a corresponding ligand partner R1

11. Procedure according to claims 10 **characterised by** the analyte being a DNA strand and the ligand R1 a DNA strand corresponding to a labelled molecular probe capable of binding to the analyte DNA strand

12. Procedure according to claims 5 to 11 **characterised by** a large number of different ligands but prepared in an identical manner as R1 and R3, are associated in the same solution and which is placed after the sample and in which an analyte is to, be detected amongst the multitude of analytes corresponding to the multitude of ligands associated

13. Procedure according to claim 12 **characterised by** the presence if at least one analyte in the sample provides a signal and the signal is proportional to the number of analytes detected in the sample

14. Procedure according to claims 5 to 6 **characterised by** an important amplification of the signal providing a sensitivity at the level of molecular for all analytes

15. Procedure according to claim 14 **characterised** as being capable of detecting substances produced in malignant pathologies attributed to a gene mutation, such as cancers, and in particular colon cancer, Alzheimer's disease and heart attack. i.

## Patentansprüche

1. Nachweis-Einheit für einen Analyten in einem Flüssig- oder Festmedium, bestehend aus
i) einer Einrichtung vakuumverpackt und gekennzeichnet in einem lichtdichten Beutel, hermetisch verschlossen und ein Trocknungsmittel enthaltend
ii) eine Menge einer Flüssgkeit eines Liganden gekoppelt mit den Gruppen R1 und R3, charakterisiert dadurch dass der Ligand an einer seiner Extremität die Gruppe R1 trägt, nicht mit einem Label versehen und ohne enzymatische Aktivität ist und mit der Fähigkeit mit R2 zu reagieren, wogegen R1 mit seinem Analyten gebunden ist oder nicht, und an der andere Extremität, und an der anderen Extremität mit einem Reagenz R3, ebenfalls nicht mit einem Label versehen und ohne enzymatische Aktivität
iii) eine Pipette zur Entnahme der Probe, und um sie direkt mit der Liganden-Lösung gekoppelt mit R1 und R3 zu mischen
die Einrichtung besteht aus
a) ein porösen Filter (1) auf dem über die komplette Länge oder suzessiv folgende Komponenten platziert sind:
i) das Reagenz R2 mit der Fähigkeit einen Liganden, der R1 und R3 trägt, abzufangen mittels einer Interaktion von R1 und R2, je nachdem ob R 1 mit seinem Analyten gekoppelt ist oder nicht, und
ii) nachgelagert der vorausgehenden Zone Reagenz R4 gekoppelt mit einem oder mehreren enzymatischen nachweis-Molekülen und fähig, einen Liganden R1-R3 durch eine Interaktion mit R4 und R5 abzufangen, wobei das Reagenz R4 in einer Art und Weise platziert ist, dass es nicht immobilisiert ist, und
iii) nachgelagert dem vorherigen Bereich Komponenten mit der Fähigkeit mit den R4-gekoppelten enzymatischen Label zu interagieren, in einer Art und Weise, dass zahlreiche Molekule pro Sekunde von einer Substanz X1 an der Extremität von diesem Filter produziert werden, die den Signalbereich darstellt, der die Ankunft oder nicht-Ankunft des Komplex-Liganden ader Analyten anzeigt.
iv) eine obere Komponente, mit einem Loch versehen (5), oberhalb der Position von R2 auf dem Filter, die die Plazierung einer Proobe auf den Filter erlaubt, bevor die Ligandenlösung an derselben Position aufgebracht wird.
b) ein Trichter (7) oberhalb des Loches (5) zur Hilfe der Deponierung von den Probe und Ligandenlösungen
c) ein Mittelteil aus einem starren Material perforiert mit einem rechteckigen Loch von der Größe des Filters, das das Auffalten der Filterränder verhindert, wenn das obere und untere Element eingesetzt wird.
d) ein unteren Element vom selben starren Material aber mit einem Loch (3) oder einem viereckiges Fenster am Ende des unteren Teils des Filters, geschützt von einer dünnen durchsichtigen Folie um den Signalbereich von der Luft zu schützen, dort wo das Signal aus den Reaktionsprodukten sichtbar oder messbar ist.
der Filter ist zwischen den drei Elementen a)b) und c) eingeschlossen.

2. Nachweis-Einheit entsprechend Anspruch 1 charakterisiert dadurch dass sie identisch ist unabhängig von der untersuchten Komponenten es sich, mit Ausnahme der Lösung vom R1 und R3-gekoppelten Liganden, wobei die R1- Gruppe spezifisch für den untersuchten Analyten ist.

3. Nachweis-Einheit nach Ansprüchen 1 und 2 charakterisiert dadurch dass die Einrichtung mit einem Gerät verbunden ist, das das Signal lesen und quantifizieren kann

4. Nachweis-Einheit nach Ansprüche 1 und 2 charakterisiert dadurch dass das R4 Reagenz mit einem Peroxydase-Label gekoppelt ist, und wo die Reagenzien Glukose oxydase, D-Glukose und Tetramethylbenzidine sukzessiv und einzeln unterhalb von R4 of der Filterkomponente der Einheit platziert wurden

5. Verfahren für den Nachweis von einer Substanz in einer flüssigen oder festen Probe, mit den folgende Schritten:
i) Platzierung eines Tropfens der Probe (5) in den Trichter (7) auf den Filter (1) auf die Nachweiseinheit nach Anspruch 1
ii) Platzierung eines Tropfens der Ligandenlösung, die den spezifischen Liganden enthält, der an seiner Extremität mit dem Reagenz R1 gekoppelt ist, ohne Label und ohne enzymatische Aktivität und in der Lage mit dem Reagenz R2 zu reagieren, während Reagenz R1 komplexiert oder nicht komplexiert ist mit seinem Analyten, und am anderen Ende das Reagenz R3, ohne Label und ohne enzymatische Aktivität.
iii) Inkubation der genannten Einheit, so dass die Proben- und Ligandenmischungden Filter hinab wandern, mit dem Ziel, dass in Abwesenheit des Analyten R1 an ein Reagenz R2 das auf den Filter der genannten Einheit immobilisiert ist, gebunden wird und auf diese Art der Ligand auf dem Filter immobilisiert wird. In Anwesenheit des Analyten ist das Reagenz R1 des Liganden blockiert und neutralisiert und der komplexierte Ligand und Analyte wandern weiter, wo sie weiter unten auf dem Filter durch Vermittlung von R3 mit dem Reagenz R4 reagieren, das nicht auf dem Filter fixiert ist und mit einem oder mehreren enzymatischen Label-Molekülen assoziiert ist.
iv) Nachweis in dem Signalbereich am Ende vom Filter von tausenden von Molekülen pro Sekunde von einer Substanz X1 produziert durch den enzymatischen Label des Komplexes aus Analyt, dem Ligand R1-R3 und R4 gebunden an eines oder mehrere Moleküle des enzymatischen Label-Moleküls,

6. Verfahren nach Anspruch 5, charakterisiert dadurch dass der Signalbereich an der Extremität des Filters auf das das chromogene (farbstoffbildende) Substrat deponiert wurde, um mit X1 zu reagieren.

7. Verfahren nach Anspruch 6, charakterisiert durch die Produktion der Komponente X1 durch die Reaktion des Peroxidase-Labels gebunden an R4 und durch die Reagenzien a) Glukose Oxidase, b) D-Glukose und c) Tera-Methyl-Benzidine, wobei diese Reagenzien sukzessive und separat unterhalb von R4 auf den Filter platziert werden.

8. Verfahren nach Anspruch 5, charakterisiert durch eine Prozudure die einen Schritt umfasst, bevor die Probe mit einer bestimmten Menge des Liganden in Kontakt ist, wobei der Ligand weder R1 noch R3 trägt und mit einer normalen Menge des Analyten korrespondiert, mit dem Ziel, eine bestimmte Menge des Analyten im Probetropfen zu neutralisierenwie beschrieben in Schritt a) von Anspruch 5 was einen semi-quantitative Nachweis des Analyten erlaubt, wobei ein im Vergleich zum Normalen ein Signal im Überschuss produziert wird.

9. Verfahren nachdem einen von den Ansprüche 5 bis 8 charakterisiert dadurch, dass der Analyt ein Antigen und der Ligand ein für das Antigen spezifischer Antikörper ist, oder umgekehrt wobei der Analyt der Antikörper und den Ligand das Antigen ist

10. Verfahren nachdem einen der Ansprüche 5 bis 8 charakterisiert dadurch, dass der Analyt jedes Molekul sein kann, das fähig ist, an einem entsprechenden Liganden R1 zu binden

11. Verfahren nach dem Anspruch 10 in dem das der Analyt ein DANN-Fragment ist, und der Ligand R1 eine markierte molekulare Sonde ist die das gesuchte Stück DNA erkennen kann

12. Verfahren nach den Ansprüchen 5 bis 11 charakterisiert dadurch dass eine grosse Zahl von unterschiedlischen aber ähnlich wie R1 und R3 hergestellten Liganden in derselben Lösung assoziert sind, die nach nach dem Probenauftrag aufgetragen wird. In der Probe wird innerhalb der Vielzahl von zu Liganden korrespondierenden Analyten mindestens einer gesucht.

13. Verfahren nach dem Anspruch 12 charakterisiert dadurch, dass ween mindestens einer der Analyten in der Probe vorhanden ist, ein Signal erzeugt wird, welches auch zur Anzahl der verschiedenen detektierten Analyten proportional ist.

14. Verfahren nach den Ansprüche 5 und 6 charakterisiert dadurch, dass die erhabliche Verstärkung des erhaltenen Signals eine Nachweisempfindlichkeit von allen Analyten im molekularem Bereich erlaubt

15. Verfahren nach dem Anspruch 14 charakterisiert dadurch dass das es einen Nachweis erlaubt von Substanzen produziert durch krebserregende Krankheiten, die durch eine genetische Mutation oder Translation verursacht sind wie Krebs, ins besondere dar Kolonkrebs, Alzheimer, und Herzinfarkt
